# EUROPÄISCHE PATENTSCHRIFT

(11) **EP 1 383 723 B1**
(45) Veröffentlichungstag und Bekanntmachung des Hinweises auf die Patenterteilung: **25.01.2006**
(21) Anmeldenummer: 02724288.2
(22) Anmeldetag: 17.04.2002
(51) Int. Cl.: C07C 17/42, C07C 17/16, C07C 21/22

(54) **VERFAHREN ZUM EIGENSICHEREN UMGANG MIT 3-CHLORPROPIN**
METHOD FOR THE INTRINSICALLY SAFE HANDLING OF 3-CHLORPROPYNE
PROCEDE DE MANIPULATION INTRINSEQUEMENT SURE DE 3-CHLOROPROPYNE

(30) Priorität: 21.04.2001 DE 10119720
(43) Veröffentlichungstag der Anmeldung: 28.01.2004
(73) Patentinhaber: BASF Aktiengesellschaft, 67056 Ludwigshafen (DE)
(72) Erfinder: STAMM, Armin, 55268 Nieder-Olm (DE); KNEUPER, Heinz-Josef, 67150 Niederkirchen (DE); RITTINGER, Stefan, 25050 Kuantan, Pahang (MY); DRANSFELD, Peter, 67308 Albisheim (DE); SCHILDBERG, Hans-Peter, 67433 Neustadt (DE); HEILIG, Manfred, 67346 Speyer (DE); WEBER, Theodor, 67063 Ludwigshafen (DE)
(86) Internationale Anmeldenummer: PCT/EP2002/004249
(87) Internationale Veröffentlichungsnummer: WO 2002/085824

(56) Entgegenhaltungen:
- DE-A- 19 810 036
- GB-A- 1 132 417
- US-A- 2 926 204
- PATENT ABSTRACTS OF JAPAN vol. 2000, no. 11, 3. Januar 2001 (2001-01-03) & JP 2000 219644 A (SUMITOMO CHEM CO LTD), 8. August 2000 (2000-08-08)

## Beschreibung

Die vorliegende Erfindung betrifft ein Verfahren zum eigensicheren Umgang mit 3-Chlorpropin, insbesondere zu dessen Lagerung, zu dessen Transport sowie zu dessen Herstellung, in Gegenwart eines Verdünnungsmittels mit einem Siedepunkt unter Normaldruck im Bereich von -50°C (223 K) bis 200°C (473 K). Die vorliegende Erfindung betrifft ferner die Verwendung eines derart gelagerten, transportierten und/oder hergestellten 3-Chlorpropins in der Synthese von Farbstoffen, Pharma- und Agrarwirkstoffen, Galvanohilfsmitteln, Desinfektionsmitteln, Steroiden und Wuchshormonen.

3-Chlorpropin (Propargylchlorid) ist ein wichtiges Zwischenprodukt bei der Synthese einer Vielzahl chemischer Produkte, insbesondere von Farbstoffen, Pharma- und Agrarwirkstoffen, Galvanohilfsmitteln, Desinfektionsmitteln, Steroiden und Wuchshormonen.

Aliphatische Chlorverbindungen werden im Allgemeinen durch Umsetzung der entsprechenden Alkohole mit einem Chlorierungsmittel in Gegenwart eines Katalysators hergestellt. Für die Herstellung von 3-Chlorpropin im technischen Maßstab ist vor allem die Umsetzung von Propin-3-ol (Propargylalkohol) mit Phosgen (COCl₂) oder Thionylchlorid (SOCl₂) in Gegenwart von Katalysatoren geeignet. Als Koppelprodukte entstehen dabei nur die gasförmigen Produkte Chlorwasserstoff, Kohlendioxid beziehungsweise Schwefeldioxid, die aus dem Reaktionsgemisch entweichen.

In EP-A 0 786 442 ist ein zweistufiges Verfahren zur Herstellung von Alkylchloriden aus den entsprechenden Alkoholen beschrieben. In der ersten Stufe erfolgt eine Umsetzung mit Chlorwasserstoff und in der zweiten Stufe mit Phosgen in Gegenwart von Hexaalkylguanidiniumhalogeniden, Halogeniden quartärer Ammonium- und Phosphoniumverbindungen oder Pyridiniumhalogeniden als Katalysator bei einer Temperatur von 80 bis 160°C.

EP-A 0 375 920 lehrt die Herstellung von Alkyl- und Alkenylchloriden durch Decarboxylierung der entsprechenden Alkyl- oder Alkenyl-chlorformiate in Gegenwart quartärer Ammonium- oder Phosphoniumsalze als Katalysator bei einer Temperatur von 50 bis 200°C und üblicherweise bei 90 bis 170°C. Die Herstellung der Alkyl- oder Alkenyl-chlorformiate erfolgt durch Umsetzung der entsprechenden Alkohole mit Phosgen in einer vorgeschalteten Synthesestufe oder in-situ.

Die DE-Auslegeschrift 1 135 893 beschreibt die Synthese von Propargylchlorid durch Umsetzung von Propargylalkohol mit Phosgen in Gegenwart N,N-dialkylsubstituierter Carbonsäureamide oder N-alkylsubstituierter Lactame als Katalysator. Bei dem Verfahren wird der flüssige Katalysator mit Phosgen gesättigt und anschließend Propargylalkohol und weiteres Phosgen eingeleitet.

In EP-A 0 514 683 und EP-A 0 645 357 sind Verfahren zur Herstellung von Alkinylchloriden durch Umsetzung der entsprechenden Alkohole mit Phosgen oder Thionylchlorid in Gegenwart eines Katalysators offenbart. Nach EP-A 0 514 683 wird ein flüssiges Phosphinoxid mit dem Chlorierungsmittel versetzt und anschließend der entsprechende Alkohol und weiteres Chlorierungsmittel zugegeben. Nach EP-A 0 645 357 wird durch Einleiten von Chlorwasserstoff in N,N-disubstituiertem Formamid zunächst das Katalysator-Addukt hergestellt, anschließend der entsprechende Alkohol zugegeben und das Chlorierungsmittel eingeleitet.

Die Offenlegungsschrift WO 99/46226 beschreibt ein kontinuierliches Verfahren zur Herstellung von Propargylchlorid durch Umsetzung von Propargylalkohol mit einem Chlorierungsmittel in Gegenwart eines Katalysators und in Gegenwart eines substituierten aromatischen Kohlenwasserstoffs als Verdünnungsmittel in einer Menge von 10 bis 50 Gew.-%, bezogen auf die eingesetzte Menge an Propargylalkohol.

JP-2000219644 offenbart ein Verfahren zur sicheren Destillation von 3-Chloropropin, bei dem man das 3-Chloropropin in Gegenwart von Methyl-tert.-butyl-ether destilliert, wobei überall in der Destillationsvorrichtung der Gehalt an Methyl-tert.-butyl-ether nicht weniger als 25 Gew.-% beträgt.

Aus der Literatur ist bekannt, daß 3-Chlorpropin sowohl in der kondensierten Phase als auch in der Gasphase zu einem selbständig fortschreitenden Zerfall auch unter Ausschluß von Sauerstoff neigt (Deflagration). So beschreiben D.R. Forshey et al. in Fire Technology 5 (1969) Seite 100 bis 111 die Deflagrationsempfindlichkeit von reinem, flüssigem 3-Chlorpropin. Erst durch Zusatz von 10 Gew.-% Toluol wird eine bis etwa 600 psig.(etwa 4,3 MPa abs) deflagrationsstabile Flüssigkeit erhalten. Reines, gasförmiges 3-Chlorpropin ist bei 25°C bis hinab zu einem Druck von 0,58 psia (4 kPa abs) deflagrationsempfindlich. Ein Zusatz von 25 Vol. -% Propan erhöht die Deflagrationsgrenze auf Atmosphärendruck (0,1 MPa abs) und 48°C.

Erfindungsgemäß wurde erkannt, daß die beschriebenen Verfahren ein hohes Sicherheitsrisiko durch die Gegenwart mindestens einer deflagrationsfähigen Phase (Flüssigphase und/oder Gasphase) aufweisen und bei einer Zündung, beispielsweise durch statische Entladung, ein erheblicher Personen- und Sachschaden entstehen kann. Bei dem Verfahren nach der Lehre der DE-Auslegeschrift 1 135 893 ist aufgrund der sich einstellenden hohen Konzentrationen an 3-Chlorpropin sowohl die Flüssigphase als auch die Gasphase deflagrationsfähig. Bei dem Verfahren nach der Lehre der WO 99/46226 wird zwar aufgrund des eingesetzten substituierten aromatischen Kohlenwasserstoffs als Verdünnungsmittel eine deflagrationsstabile Flüssigphase erhalten, die im thermodynamischen Gleichgewicht stehende Gasphase ist jedoch eindeutig deflagrationsfähig.

Es bestand daher die Aufgabe, ein Verfahren zum Umgang, insbesondere zur Herstellung, zur Lagerung und zum Transport von 3-Chlorpropin zu finden, welches die oben genannten Nachteile nicht mehr besitzt, verfahrenstechnisch einfach und kostengünstig durchzuführen ist und auch in Gegenwart potentieller Zündcluellen, kein Deflagrationsrisiko aufweist. Demgemäß wurde ein Verfahren zum eigensicheren Umgang mit 3-Chlorpropin in Gegenwart eines Verdünnungsmittels mit einem Siedepunkt unter Normaldruck im Bereich von -50°C (223 K) bis 200°C (473 K) gefunden, das dadurch gekennzeichnet ist, daß man die Konzentration an 3-Chlorpropin in der Flüssigphase und in der Gasphase durch Art und Menge des Verdünnungsmittels, durch die Temperatur und durch den Gesamtdruck des Systems unterhalb der deflagrationsfähigen Konzentrationen hält.

Der Umgang mit 3-Chlorpropin, bei dem das Kriterium der Deflagrationsstabilität der Flüssigphase und der Gasphase bei einer gegebenen Temperatur und einem gegebenen Gesamtdruck des Systems erfüllt ist, wenn sich beide Phasen im thermodynamischen Gleichgewicht befinden, wird als eigensicherer Umgang bezeichnet.

Unter einer deflagrationsfähigen Konzentration ist eine Konzentration an 3-Chlorpropin in der jeweils betrachteten Phase zu verstehen, bei der durch den Einsatz einer Zündquelle eine Deflagration, das heißt ein selbständig fortschreitender Zerfall an 3-Chlorpropin, ausgelöst werden kann.

Die Bestimmung der Deflagrationsfähigkeit der Flüssigphase erfolgt in Anlehnung an den Deflagrationstest "Test C.2", welcher in "Recommendations on the Transport of Dangerous Goods - Manual of Tests and Criteria", 3^{rd} revised edition, United Nations, New York and Geneva 1999, ISBN 92-1-139068-0 auf Seite 225 bis 228 unter Abschnitt 23.4.2 beschrieben ist. Die Bestimmung wird in einem 300 mL Dewar-Gefäß mit einem inneren Durchmesser von 48 ±1 mm, welches bis etwa 20 mm unterhalb des Randes mit der zu messenden Flüssigkeit gefüllt ist, bei der gewünschten Temperatur und dem gewünschten Druck, durchgeführt. Als Zündquelle für die Flüssigphase ist eine Gasflamme mit einer Mindestlänge von 20 mm einzusetzen, welche auf die Flüssigkeitsoberfläche gerichtet wird. Die durch die Zündung eventuell ausgelöste Deflagration wird über die Ausbreitungsgeschwindigkeit der ausgelösten Temperaturwelle bestimmt. Hierzu befindet sich im Dewar-Gefäß in einem Abstand von 50 und 100 mm unterhalb des Randes jeweils ein Thermoelement, wobei die Temperaturen über den zeitlichen Verlauf hinweg festgehalten werden. Liegt die gemessene Ausbreitungsgeschwindigkeit der Temperaturwelle bei ≥ 0,35 mm/s, so gilt die Flüssigphase als deflagrationsfähig. Liegt der Wert bei < 0,35 mm/s, so gilt die Flüssigphase als nicht-deflagrationsfähig.

Die Bestimmung der Deflagrationsfähigkeit der Gasphase erfolgt nach der künftigen europäischen Norm prEN1839, Method B "bomb method" (Arbeitsdokument der Physikalisch Technischen Bundesanstalt Braunschweig, Titel "CEN/TC 305/WG1/SG 4", Stand Januar 2000) in einem zylindrischen oder sphärischen Behälter mit einem Gasphasenvolumen von mindestens 5 L, wobei beim Einsatz eines zylindrischen Behälters der Durchmesser mindestens 80 mm beträgt. Als Zündquelle für die Gasphase ist ein bei der Zündung schmelzender Platin-Draht zwischen zwei Metallstangen einzusetzen. Die Metallstangen sollten aus rostfreiem Stahl bestehen und parallel zueinander angeordnet sein. Ihre Enden sollten einen Abstand von 5 ±1 mm aufweisen und sich mittig im Behälter befinden. Der Platin-Draht sollte einen Durchmesser von 0,05 bis 0,2 mm besitzen. Spannung und Strom sind derart einzustellen, daß eine Zündenergie von 10 bis 100 J resultiert. Die durch die Zündung eventuell ausgelöste Deflagration ist über den zeitlichen Anstieg des Behälterinnendruckes zu detektieren. Als Kriterium für eine Deflagration wird der maximale, nach der Zündung im Behälter gemessene absolute Druck herangezogen. Beträgt dieser mehr als das 1,05-fache des Anfangsdrucks vor der Zündung, so gilt das Gasgemisch als deflagrationsfähig. Liegt dieser beim 1,05-fachen Wert des Anfangsdrucks vor der Zündung oder darunter, so gilt das Gasgemisch als nicht-deflagrationsfähig.

Die Deflagrationsfähigkeit der Flüssigphase und der Gasphase ist entscheidend von deren Zusammensetzung, deren Temperatur und deren Gesamtdruck abhängig. Wie allgemein bekannt und in der Literatur beschrieben, ist reines 3-Chlorpropin sowohl in der Flüssigphase als auch in der Gasphase über einen breiten Temperatur- und Druckbereich hinweg deflagrationsfähig.

Wesentlich beim erfindungsgemäßen Verfahren ist, daß man die Konzentration an 3-Chlorpropin sowohl in der Flüssigphase als auch in der Gasphase durch Art und Menge des Verdünnungsmittels, durch die Temperatur und durch den Gesamtdruck des Systems unterhalb der deflagrationsfähigen Konzentrationen hält und somit sowohl die Flüssigphase als auch die Gasphase deflagrationsstabil sind.

Durch Einstellung des thermodynamischen Gleichgewichts zwischen der Flüssigphase und der darüberstehenden Gasphase wird die Konzentration an 3-Chlorpropin derart geregelt, daß sie in beiden Phasen jeweils unterhalb der deflagrationsfähigen Konzentration liegt. Aus rein thermodynamischen Gründen ist es an keiner räumlichen Stelle des Systems möglich, eine Konzentration an 3-Chlorpropin zu erhalten, welche oberhalb der deflagrationsfähigen Konzentration liegt. So wird beispielsweise aus der Flüssigphase maximal nur soviel 3-Chlorpropin nachgeliefert, daß keine deflagrationsfähige Phase erhalten werden kann.

Im Gegensatz dazu würde bei einem nicht-erfindungsgemäßen Vorgehen durch eine unzureichende Zugabe eines Verdünnungsmittels nach Einstellung des thermodynamischen Gleichgewichts mindestens eine Phase deflagrationsfähig sein, da die Konzentration an 3-Chlorpropin oberhalb der deflagrationsfähigen Konzentration liegt. So wird, wie bereits einleitend beschrieben, bei einem Verfahren nach der Lehre der WO 99/46226 zwar eine deflagrationsstabile Flüssigphase, nicht jedoch eine deflagrationsstabile Gasphase erhalten, da aufgrund des thermodynamischen Gleichgewichts eine derart hohe Menge an 3-Chlorpropin aus der Flüssigphase nachgeliefert wird, daß die erreichte Konzentration in der Gasphase oberhalb der deflagrationsfähigen Konzentration liegt. Eine zusätzliche und kontinuierliche oder intermittierende Zufuhr einer weiteren gasförmigen Komponente, wie beispielsweise eine kontinuierliche oder intermittierende Spülung mit einem Inertgas (z.B. Stickstoff) oder eine kontinuierliche oder intermittierende Bildung von Reaktionsabgasen (z.B. Kohlendioxid und Chlorwasserstoff bei der Herstellung von 3-Chlorpropin aus Propin-3-ol und Phosgen in Gegenwart eines Katalysators) würde zwar durch Störung des thermodynamischen Gleichgewichts die Konzentration an 3-Chlorpropin in der Gasphase eventuell unterhalb der deflagrationsfähigen Konzentration halten können, jedoch nicht im Sinne eines eigensicheren Umgangs im Rahmen der vorliegenden Erfindung. So würde sich beispielsweise bei einem Versiegen der Zufuhrquelle der weiteren gasförmigen Komponente das thermodynamische Gleichgewicht einstellen und somit auch die Konzentration an 3-Chlorpropin über die deflagrationsfähigen Konzentration ansteigen.

Beim erfindungsgemäßen Verfahren ist es möglich und in der Regel sogar vorteilhaft, daß die Summe der Partialdrücke von 3-Chlorpropin und des Verdünnungsmittels in der Gasphase aufgrund des thermodynamischen Gleichgewichts kleiner ist als der gewünschte Gesamtdruck des Systems. In diesem Fall wird die Druckbilanz zwischen dem gewünschten Gesamtdruck des Systems und den Partialdrücken von 3-Chlorpropin und des Verdünnungsmittels durch die Gegenwart mindestens einer weiteren gasförmigen Komponente ausgeglichen. Durch diese vorteilhafte Option gewinnt das erfindungsgemäße Verfahren einen zusätzlichen Freiheitsgrad in der Art, daß sowohl die Temperatur als auch der Gesamtdruck des Systems bei vorgegebener Art und Menge des Verdünnungsmittels variierbar sind. Ohne eine weitere gasförmige Komponente wäre bei vorgegebener Temperatur der Gesamtdruck des Systems, und bei vorgegebenen Gesamtdruck des Systems die Temperatur thermodynamisch festgelegt.

Da alle vier genannten Parameter (Art des Verdünnungsmittels, Menge des Verdünnungsmittels, Temperatur und Gesamtdruck des Systems) einen entscheidenden Einfluß auf die Deflagrationsfähigkeit der beiden Phasen ausüben, sind sie sorgfältig festzulegen. Sofern keine Daten, beispielsweise in Form von Phasendiagrammen und Deflagrationsdiagrammen, vorliegen, sind diese im allgemeinen experimentell zu bestimmen. Bei der Festlegung der Parameter sowie der Bestimmung der erforderlichen Daten geht man in der Praxis im Allgemeinen wie folgt vor.

Aufgrund praktischer Erwägungen ist in der Regel der Parameterbereich für die Temperatur und den Gesamtdruck des Systems vorgegeben. Im Allgemeinen wird man mit 3-Chlorpropin in einem Temperaturbereich von -50°C (223 K) bis 200°C (473 K) und bei einem Gesamtdruck von 0,01 bis 5 MPa abs umgehen. Als Startwert für die folgenden Bestimmungen wählt man vorteilhafterweise einen Temperaturwert und einen Gesamtdruckwert aus, bei dem mit dem 3-Chlorpropin wunschgemäß umgegangen werden soll. Des weiteren ist das gewünschte Verdünnungsmittel (vorstehend "Art des Verdünnungsmittels" genannt) auszuwählen. Relevante Kriterien zur Wahl des Verdünnungsmittels sind an späterer Stelle beschrieben.

Nachdem nun Temperatur, Gesamtdruck des Systems und die Art des Verdünnungsmittels feststehen, wählt man die für die folgenden Bestimmungen der Daten gewünschte Menge des Verdünnungsmittels und realisiert experimentell ein entsprechendes System. Sofern die Gegenwart mindestens einer weiteren Komponente in der Gasphase zur Einstellung des gewünschten Gesamtdrucks erforderlich ist (siehe oben), ist diese dem System zuzufügen. Das experimentell realisierte System soll möglichst praxisnah alle Komponenten des späteren technischen Systems, beispielsweise Nebenprodukte oder Katalysatoren enthalten. Handelt es sich um die Herstellung von 3-Chlorpropin, so ist es im Allgemeinen vorteilhaft, diese für die Bestimmung der Daten möglichst praxisnah experimentell zu realisieren.

Die Bestimmung der Deflagrationsfähigkeit der Flüssigphase erfolgt vorteilhafterweise durch die Entnahme einer Probe und die Durchführung der Messung der Deflagrationsfähigkeit der Flüssigphase wie oben beschrieben (unter Anlehnung an den Deflagrationstest "Test C.2", "Recommendations on the Transport of Dangerous Goods - Manual of Tests and Criteria", 3^{rd} revised edition, United Nations, New York and Geneva 1999, ISBN 92-1-139068-0, Seite 225 bis 228, Abschnitt 23.4.2). Alternativ kann für die Messung der Deflagrationsfähigkeit der Flüssigphase auch eine separat hergestellte Mischung eingesetzt werden, welche die gleiche Zusammensetzung wie die zu messende Flüssigphase aufweist.

Die Bestimmung der Deflagrationsfähigkeit der Gasphase erfolgt vorteilhafterweise durch die Entnahme einer Probe und die Durchführung der Messung der Deflagrationsfähigkeit der Gasphase wie oben beschrieben (Norm prEN1839, Method B "bomb method", Arbeitsdokument der Physikalisch Technischen Bundesanstalt Braunschweig, Titel "CEN/TC 305/WG1/SG 4", Stand Januar 2000). Alternativ kann für die Messung der Deflagrationsfähigkeit der Gasphase auch eine separat hergestellte Mischung eingesetzt werden, welche die gleiche Zusammensetzung wie die zu messende Gasphase aufweist. Es ist ebenso möglich, die Zusammensetzung der Gasphase über gemessene oder tabellierte Daten des thermodynamischen Gleichgewichts zwischen Gas- und Flüssigphase aus der gewählten Flüssigphasenzusammensetzung zu ermitteln.

Das Ergebnis obiger Messungen gibt eine Aussage darüber, ob unter den gewählten Bedingungen die Flüssigphase und/oder die Gasphase deflagrationsfähig ist/sind oder nicht. Ihm liegt ein konkreter Satz von Parametern zugrunde, ohne deren Einflüsse auf die Deflagrationsfähigkeit bei kleineren oder größeren Abweichungen, insbesondere der Lage der Deflagrationsgrenze zu kennen. Unter Deflagrationsgrenze ist dabei der Übergang zwischen deflagrationsfähig und nicht-deflagrationsfähig unter Variation mindestens eines Parameters, wie beispielsweise die Zusammensetzung der Flüssig- oder Gasphase, die Temperatur oder den Gesamtdruck des Systems zu verstehen.

Um auch eine Aussage über die Deflagrationsfähigkeit bei kleineren oder größeren Abweichungen der eingestellten Parameter zu erhalten, ist es daher vorteilhaft, einzelne Parameter zu variieren und weitere Bestimmungen zur Deflagrationsfähigkeit durchzuführen. Bei der Herstellung von 3-Chlorpropin ist in diesem Sinne auch der Reaktionsfortgang als Parameter zu verstehen, da sich die Zusammensetzung der Flüssigphase und der Gasphase im Laufe der Reaktion ändern kann.

Das Ergebnis derartiger Messungen ergibt schließlich eine Über-sicht der Deflagrationssensitivität des Systems bei Variation der Parameter. Es ermöglicht eine Aussage darüber, ob das System auch unter Zugrundelegung der zu erwartenden Schwankungen der Parameter weiterhin eigensicher ist oder nicht. Zur Realisierung einer Sicherheitsreserve ist es von Vorteil, die Konzentration an 3-Chlorpropin in der Flüssigphase und in der Gasphase derart einzustellen, daß innerhalb der erwarteten Schwankungsbreite der Parameter die Grenze zu einem deflagrationsfähigen System nicht erreicht wird.

Die beim erfindungsgemäßen Verfahren eingesetzten Verdünnungsmittel weisen einen Siedepunkt unter Normaldruck im Bereich von -50°C (223 K) bis 200°C (473 K) auf. Bei den Verdünnungsmitteln kann es sich um organische und anorganische Verbindungen handeln. Sie sollten gegenüber 3-Chlorpropin chemisch inert sein. Wird das erfindungsgemäße Verfahren bei der Herstellung von 3-Chlorpropin eingesetzt, so sollten die Verdünnungsmittel auch gegenüber dem eingesetzten Edukt, dem Chlorierungsmittel und dem gegebenenfalls benötigten Katalysator chemisch inert sein. "chemisch inert" heißt, daß sich die Verdünnungsmittel unter den gewählten Bedingungen nicht mit den genannten Stoffen chemisch umsetzen. Bevorzugt sind die eingesetzten Verdünnungsmittel unter den gewählten Bedingungen mit 3-Chlorpropin vollkommen mischbar.

Bei den im erfindungsgemäßen Verfahren einsetzbaren Verdünnungsmitteln kann es sich beispielsweise um aliphatische, aromatische oder araliphatische, gesättigte oder ungesättigte, unsubstituierte oder substituierte Kohlenwasserstoffe handeln. Die Kohlenwasserstoffe können ein oder mehrere Heteroatome, wie etwa Sauerstoff, Stickstoff, Schwefel oder Phosphor, wie beispielsweise -O-, -S-, -SO-, -SO₂-, -NH-, -NR-, -CO-, -PR-, -P(O)R- und/oder -N= in aliphatischen oder aromatischen Systemen, enthalten. Sie können ferner durch eine oder mehrere funktionelle Gruppen, welche beispielsweise Sauerstoff, Stickstoff, Schwefel und/oder Halogen enthalten, wie beispielsweise -COOR, -F, -Cl, -Br, -I und/oder -CN, substituiert sein. Als anorganisches Verdünnungsmittel sei Schwefeldioxid oder unter Druck flüssiges Kohlendioxid genannt. Das erfindungsgemäße Verfahren kann auch mit einem Gemisch verschiedener Verdünnungsmittel durchgeführt werden.

Als Beispiele der bevorzugten Verdünnungsmittel seien
- unverzweigte und verzweigte C₃- bis C₁₁-Alkane, wie beispielsweise Propan, n-Butan, Isobutan (2-Methylpropan), n-Pentan, 2-Methylbutan (Isopentan), 2,2-Dimethylpropan, n-Hexan, 2-Methylpentan, 3-Methylpentan, 2,3-Dimethylbutan, 2,2-Dimethylbutan, n-Heptan, isomere Heptane, n-Octan, isomere Octane, n-Nonan, isomere Nonane, n-Decan, isomere Decane, n-Undecan und isomere Undecane;
- unverzweigte und verzweigte C₅- bis C₁₂-Cycloalkane mit einem oder zwei, gegebenenfalls kondensierten 5- bis 8-Ringen, wie beispielsweise Cyclopentan, Cyclohexan, Cycloheptan, Cyclooctan, Methylcyclopentan, Methylcyclohexan und Decahydronaphthalin (Decalin);
- Benzol und C₁- bis C₄-alkylsubstituierte Benzole mit insgesamt 7 bis 10 Kohlenstoffatomen, wie beispielsweise Toluol, o-Xylol, m-Xylol, p-Xylol, Ethylbenzol, 1,3,5-Trimethylbenzol (Mesitylen), 1,2,4-Trimethylbenzol, 2-Ethyltoluol, 3-Ethyltoluol, 4-Ethyltoluol, Propylbenzol, Isopropylbenzol (Cumol), 1,2,4,5-Tetramethylbenzol, 1,2-Diethylbenzol, 1,4-Diethylbenzol, Butylbenzol, Isobutylbenzol (2-Methyl-1-phenylpropan), sek.-Butylbenzol (2-Phenylbutan) und tert.-Butylbenzol (2-Methyl-2-phenylpropan) ;
- aliphatische oder araliphatische Ether mit insgesamt 2 bis 10 Kohlenstoffatomen, wie beispielsweise Dimethylether, Diethylether, Methyl-tert.-butylether, Dipropylether, Diisopropylether, n-Butylethylether, Ethyl-tert.-butylether, Methyl-tert.-pentylether, Dibutylether, Dipentylether, 1,2-Dimethoxyethan (Ethylenglycoldimethylether), 1,2-Diethoxyethan (Ethylenglycoldiethylether), Bis-(2-methoxyethyl)-ether (Diethylenglycoldimethylether), Bis-(2-ethoxyethyl)-ether (Diethylenglycoldiethylether), Tetrahydrofuran, 1,4-Dioxan und Anisol (Methoxybenzol);
- aliphatische oder araliphatische Ester mit insgesamt 2 bis 10 Kohlenstoffatomen, wie beispielsweise Ameisensäuremethylester (Methylformiat), Ameisensäureethylester (Ethylformiat), Ameisensäurepropylester (Propylformiat), Ameisensäurebutylester (Butylformiat), Ameisensäurepentylester (Pentylformiat), Ameisensäurehexylester (Hexylformiat), Ameisensäure-2-ethylhexylester (2-Ethylhexyl-formiat), Essigsäuremethylester (Methylacetat), Essigsäureethylester (Ethylacetat), Essigsäurepropylester (Propylacetat), Essigsäurebutylester (Butylacetat), Essigsäurepentylester (Pentylacetat), Essigsäurehexylester (Hexylacetat), Essigsäure-2-ethylhexylester (2-Ethylhexyl-acetat), Propansäuremethylester (Methylpropionat), Propansäureethylester (Ethylpropionat), Propansäurepropylester (Propylpropionat), Propansäurebutylester (Butylpropionat), Propansäurepentylester (Pentylpropionat), Propansäurehexylester (Hexylpropionat), Butansäureme-thylester (Methylbutyrat), Butansäureethylester (Ethylbutyrat), Butansäurepropylester (Propylbutyrat), Butansäurebutylester (Butylbutyrat), Butansäurepentylester (Pentylbutyrat) und Butansäurehexylester (Hexylbutyrat) ;
- halogenierte aliphatische, aromatische oder araliphatische Kohlenwasserstoffe mit 1 bis 10 Kohlenstoffatomen, wie beispielsweise Chlormethan, Dichlormethan, Trichlormethan, Tetrachlormethan, 1,2-Dichlorethan und Chlorbenzol, bevorzugt mit 1 bis 3 Kohlenstoffatomen, wie beispielsweise Chlorme-5 than, Dichlormethan, Trichlormethan und 1,2-Dichlorethan;

oder deren Gemische genannt.

Das Verdünnungsmittel setzt man beim erfindungsgemäßen Verfahren im Allgemeinen in einer Menge von 1 bis 2000 Gew.-% in der Flüssigphase, bezogen auf die Menge an 3-Chlorpropin, ein.

In einer der bevorzugten Variante des erfindungsgemäßen Verfahrens setzt man ein gegenüber 3-Chlorpropin gleich oder höher siedendes Verdünnungsmittel ein. Der Siedepunkt von 3-Chlorpropin beträgt nach Literaturangeben unter Normaldruck etwa 57°C. Die obere Grenze des Siedepunktes des genannten Verdünnungsmittels entspricht den bereits genannten 200°C (473 K). Besonders bevorzugt setzt man ein Verdünnungsmittel ein, welches einen Siedepunkt unter Normaldruck im Bereich von dem des 3-Chlorpropins bis 150°C (423 K) aufweist.

Das gegenüber 3-Chlorpropin gleich oder höher siedende Verdünnungsmittel dieser bevorzugten Variante setzt man bevorzugt in einer Menge von 50 bis 2000 Gew.-%, besonders bevorzugt von 50 bis 1000 Gew.-% in der Flüssigphase, bezogen auf die Menge an 3-Chlorpropin in der Flüssigphase, ein.

Als gegenüber 3-Chlorpropin gleich oder höher siedende Verdünnungsmittel setzt man bei dieser bevorzugten Variante des erfindungsgemäßen Verfahrens besonders bevorzugt
- ein unverzweigtes oder verzweigtes C₆- bis C₈-Alkan, wie beispielsweise n-Hexan, 2-Methylpentan, 3-Methylpentan, 2,3-Dimethylbutan, 2,2-Dimethylbutan, n-Heptan, isomere Heptane, n-Octan und isomere Octane, insbesondere n-Hexan, 2-Methylpentan und isomere Heptane;
- ein unverzweigtes oder verzweigtes C₆- bis C₈-Cycloalkan mit einem 5- oder 6- Ring, wie beispielsweise Cyclopentan, Cyclohexan, Cycloheptan, Cyclooctan, Methylcyclopentan und Methylcyclohexan, insbesondere Cyclopentan und Cyclohexan;
- Benzol, Toluol, Ethylbenzol, Xylole (konkret o-Xylol, m-Xylol und p-Xylol), insbesondere Toluol;

oder Gemische davon ein.

In einer weiteren der bevorzugten Variante des erfindungsgemäßen Verfahrens setzt man eine Mischung aus (a) einem gegenüber 3-Chlorpropin gleich oder höher siedenden Verdünnungsmittel und (b) einem gegenüber 3-Chlorpropin niedriger siedenden Verdünnungsmittel ein. Der Siedepunkt von 3-Chlorpropin beträgt nach Literaturangeben unter Normaldruck etwa 57°C. Die obere Grenze des Siedepunktes des unter (a) genannten Verdünnungsmittels entspricht den bereits genannten 200°C (473 K). Die untere Grenze des Siedepunktes des unter (b) genannten Verdünnungsmittels entspricht den bereits genannten -50°C (223 K), Besonders bevorzugt setzt man als unter (a) genanntes Verdünnungsmittel ein Verdünnungsmittel ein, welches einen Siedepunkt unter Normaldruck im Bereich von dem des 3-Chlorpropins bis 150°C (423 K) aufweist.

Das gegenüber 3-Chlorpropin gleich oder höher siedende Verdünnungsmittel dieser bevorzugten Variante setzt man bevorzugt in einer Menge von 1 bis 1000 Gew.-%, besonders bevorzugt von 1 bis 100 Gew.-% und ganz besonders bevorzugt von 1 bis 20 Gew.-% in der Flüssigphase, bezogen auf die Menge an 3-Chlorpropin in der Flüssigphase, ein. Das gegenüber 3-Chlorpropin niedriger siedende Verdünnungsmittel dieser bevorzugten Variante setzt man bevorzugt in einer Menge von 1 bis 500 Gew.-%, besonders bevorzugt von 5 bis 250 Gew.-% in der Flüssigphase, bezogen auf die Menge an 3-Chlorpropin in der Flüssigphase, ein.

Was die Beispiele der bevorzugten Verdünnungsmittel der unter (a) genannten Verdünnungsmittel mit einem Siedepunkt gleich oder höher dem des 3-Chlorpropins angeht, sei auf die obige Aufzählung in der Beschreibung der zuvor genannten Variante des erfindungsgemäßen Verfahrens verwiesen.

Als gegenüber 3-Chlorpropin gleich oder höher siedende Verdünnungsmittel (a) setzt man bei dieser bevorzugten Variante des erfindungsgemäßen Verfahrens besonders bevorzugt
- ein unverzweigtes oder verzweigtes C₆- bis C₈-Alkan, wie beispielsweise n-Hexan, 2-Methylpentan, 3-Methylpentan, 2,3-Dimethylbutan, 2,2-Dimethylbutan, n-Heptan, isomere Heptane, n-Octan und isomere Octane, insbesondere n-Hexan, 2-Methylpentan, 3-Methylpentan und isomere Heptane;
- ein unverzweigtes oder verzweigtes C₆- bis C₈-Cycloalkan mit einem 5- oder 6- Ring, wie beispielsweise Cyclopentan, Cyclohexan, Cycloheptan, Cyclooctan, Methylcyclopentan und Methylcyclohexan, insbesondere Cyclopentan und Cyclohexan;
- Benzol, Toluol, Ethylbenzol, Xylole (konkret o-Xylol, m-Xylol und p-Xylol), insbesondere Toluol;

oder Gemische davon, und als gegenüber 3-Chlorpropin niedriger siedende Verdünnungsmittel (b) besonders bevorzugt
- ein unverzweigtes oder verzweigtes C₃- bis C₅-Alkan, wie beispielsweise Propan, n-Butan, Isobutan (2-Methylpropan), n-Pentan, 2-Methylbutan (Isopentan) und 2,2-Dimethylpropan, insbesondere Propan, Isobutan (2-Methylpropan) und n-Pentan;
- Cyclopentan,
- eine aliphatischen Ether mit insgesamt 2 bis 5 Kohlenstoffatomen, wie beispielsweise Dimethylether, Diethylether, Methyl-tert.-butylether, 1,2-Dimethoxyethan (Ethylenglycoldimethylether), Tetrahydrofuran und 1,4-Dioxan, insbesondere Dimethylether, Diethylether und Methyl-tert.-butylether;
- Chlormethan;

oder Gemische davon, ein.

Der kombinierte Einsatz eines gegenüber 3-Chlorpropin gleich oder höher siedenden Verdünnungsmittels mit einem gegenüber 3-Chlorpropin niedriger siedenden Verdünnungsmittel hat den Vorteil, den eigensicheren Umgang mit einer geringeren Gesamtmenge an Verdünnungsmittel sicherzustellen als beispielsweise beim Einsatz nur eines Verdünnungsmittels mit einem gegenüber 3-Chlorpropin gleichen oder höheren Siedepunkt der Fall ist. Das niedriger siedende Verdünnungsmittel setzt besonders effektiv in der Gasphase und das gleich oder höher siedende Verdünnungsmittel besonders effektiv in der Flüssigphase die Konzentration von 3-Chlorpropin ab.

Wie weiter oben bereits beschrieben, ist es beim erfindungs-gemäßen Verfahren möglich und in der Regel sogar vorteilhaft, daß die Summe der Partialdrücke von 3-Chlorpropin und des Verdünnungsmittels in der Gasphase aufgrund des thermodynamischen Gleichgewichts kleiner ist als der gewünschte Gesamtdruck des Systems und daher die Druckbilanz durch die Gegenwart mindestens einer weiteren gasförmigen Komponente ausgeglichen werden kann. Bevorzugt gleicht man die Druckbilanz zwischen dem Gesamtdruck des Systems und dem Partialdruck von 3-Chlorpropin sowie dem Partialdruck des Verdünnungsmittels durch Gegenwart eines Inertgases aus.

Unter Inertgase sind Stoffe zu verstehen, welche einen Siede-oder Sublimationspunkt unter Normaldruck von unterhalb -50°C (223 K) besitzen und gegenüber 3-Chlorpropin chemisch inert sind, das heißt unter den gewählten Bedingungen sich nicht mit 3-Chlorpropin chemisch umsetzen.

Als Beispiele geeigneter Inertgase seien Helium, Neon, Argon, Krypton, Xenon, Stickstoff, Kohlenmonoxid, Kohlendioxid, Methan, Ethan, Ethen, Chlorwasserstoff und deren Gemische genannt. Als bevorzugte Inertgase seien Methan, Stickstoff, Kohlenmonoxid, Kohlendioxid, Chlorwasserstoff und deren Gemische genannt.

Beim erfindungsgemäßen Verfahren hält man das System bevorzugt bei einer Temperatur im Bereich von -20°C (253 K) bis 100°C (373 K) und besonders bevorzugt im Bereich von 0°C (273 K) bis 100°C (373 K). Den Gesamtdruck des Systems hält man bevorzugt bei 0,05 bis 1 MPa abs und besonders bevorzugt bei 0,05 bis 0,5 MPa abs.

Eine der bevorzugten Varianten des erfindungsgemäßen Verfahrens ist, daß man das 3-Chlorpropin lagert oder transportiert. Die Zeitdauer für die Lagerung und den Transport ist bei erfindungsgemäßen Verfahren in der Regel unerheblich. Sie kann wenige Minuten bis mehrere Jahre betragen. Im Allgemeinen liegt die Zeitdauer im Bereich von einigen Stunden bis mehreren Monaten. Für die Lagerung und den Transport von 3-Chlorpropin setzt man im Allgemeinen Behälter ein. Ihre Größe ist für das erfindungsgemäße Verfahren in der Regel unerheblich. In der Regel setzt man Behälter im L- bis m³-Bereich ein.

Die Lagerung und den Transport von 3-Chlorpropin führt man bevorzugt bei einer Temperatur von 0°C (273 K) bis 100°C (373 K) und besonders bevorzugt von 0°C (273 K) bis 50°C (323 K) durch. Der Gesamtdruck des Systems beträgt bevorzugt 0,05 bis 0,5 MPa abs und besonders bevorzugt 0,09 bis 0,2 MPa abs. Insbesondere lagert und transportiert man 3-Chlorpropin bei Atmosphärendruck.

Eine weitere der bevorzugten Varianten des erfindungsgemäßen Verfahrens ist, daß man das 3-Chlorpropin durch Umsetzung von Propin-3-ol mit einem Chlorierungsmittel, gegebenenfalls in Gegenwart eines Katalysators, herstellt.

Als Chlorierungsmittel werden im Allgemeinen Phosgen, Chlorameisensäuretrichlormethylester (Diphosgen), Bis-(trichlormethyl)-carbonat (Triphosgen). Thionylchlorid oder Phosphortrichlorid eingesetzt, wobei der Einsatz von Phosgen bevorzugt ist. Phosgen kann beim erfindungsgemäßen Verfahren gasförmig oder flüssig zugegeben werden. Beim Einsatz von Phosgen, Chlorameisensäuretrichlormethylester (Diphosgen), Bis-(trichlormethyl)-carbonat (Triphosgen) und Thionylchlorid ist im Allgemeinen die Gegenwart eines Katalysators erforderlich. Beim Einsatz von Phosphortrichlorid ist die Gegenwart eines Katalysators optional.

Als Katalysatoren können beim erfindungsgemäßen Verfahren prinzipiell alle üblichen und bekannten Katalysatoren für die Chlorierung von Carbonsäuren und Alkoholen eingesetzt werden. Als Beispiele seien
- N,N-disubstituierte Formamide, insbesondere N,N-disubstituierte Formamide der allgemeinen Formel (I) 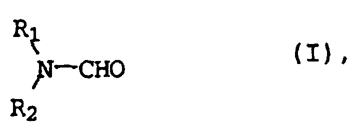 in der R¹ und R² unabhängig voneinander C₁- bis C₈-Alkyl oder R¹ und R² gemeinsam eine C₄- oder C₅-Alkylenkette, welche gegebenenfalls durch ein oder mehrere Sauerstoff- oder Stickstoff-Atome unterbrochen sein kann, bedeuten, wie beispielsweise N,N-Dimethylformamid, N,N-Diethylformamid, N,N-Dipropylformamid, N,N-Diisopropylformamid, N,N-Dibutylformamid, N,N-Di-sek.-butylformamid, N,N-Diisobutylformamid, N,N-Dipentylformamid, N,N-Dihexylformamid, N,N-Dioctylformamid, N-Formylpyrrolidin und N-Formylpiperidin,
- Tetraalkyl-substituierte Harnstoffe, insbesondere Tetra-C₁₋bis C₄-alkylsubstituierte Harnstoffe, wie beispielsweise Tetramethylharnstoff, Tetraethylharnstoff und Tetrabutylharnstoff;
- alicyclische N,N'-alkyl-- oder aryl-substituierte Harnstoffe, insbesondere alicyclische N,N'- C₁- bis C₄-alkylsubstituierte Harnstoffe mit einem 5- oder 6-Ringsystem, wie beispielsweise Dimethylethylenharnstoff (N,N'-Dimethyl-2-imidazolidinon) und Dimethylpropylenharnstoff (N,N'-Dimethyltetrahydro-2(1H)-pyrimidinon);
- Tetraalkylamidiniumsalze, insbesondere Tetra-C₁- bis C₄-alkylformamidiniumsalze, wie beispielsweise Tetramethylformamidiniumchlorid;
- Hexaalkylguanidiniumsalze, insbesondere Hexa-C₁- bis C₄-alkylguanidiniumsalze, wie beispielsweise N,N,N',N',N",N"-Hexamethylguanidiniumchlorid und N,N,N',N',N'',N"-Hexabutylguanidiniumchlorid;
- Trialkyl- oder Triarylphosphinoxide, insbesondere Tri-C₁- bis C₈-alkylphosphinoxide oder Triarylphosphinoxide, wie beispielsweise Triphenylphosphinoxid und Tri-C₆- bis C₈-alkylphosphinoxid-Gemische (z.B. "Cyanex® 923" der Firma Cytec-Industries);
- Pyridin oder dessen alkylsubstituierte Derivate, insbesondere Mono-C₁-C₄-alkylpyridine, wie beispielsweise 2-Methylpyridin (α-Picolin), 3-Methylpyridin (β-Picolin) und 4-Methylpyridin (γ-Picolin);
- N-Alkylimidazole, insbesondere N-C₁- bis C₄-Alkylimidazole, wie beispielsweise N-Methylimidazol;

genannt.

Die Menge an Katalysator beträgt im Allgemeinen 0,1 bis 20 mol-% und bevorzugt 0,5 bis 10 mol-%, bezogen auf die eingesetzte Menge an Propin-3-ol.

Die Umsetzung mit dem Chlorierungsmittel wird im Allgemeinen bei einer Temperatur von 0 bis 150°C, bevorzugt von 20 bis 100°C und ganz besonders bevorzugt von 40 bis 70°C durchgeführt. Sie erfolgt im Allgemeinen bei einem Druck von 0,01 bis 5 MPa abs, bevorzugt 0,05 bis 0,2 MPa abs, besonders bevorzugt bei 0,08 bis 0,15 MPa abs, insbesondere bei Atmosphärendruck.

Die Umsetzung mit dem Chlorierungsmittel kann diskontinuierlich oder kontinuierlich durchgeführt werden.

In einer besonders bevorzugten Variante zur Herstellung von 3-Chlorpropin nach dem erfindungsgemäßen Verfahren verwendet man als Chlorierungsmittel Phosgen und als Katalysator ein N,N-disubstituiertes Formamid der allgemeinen Formel (I) 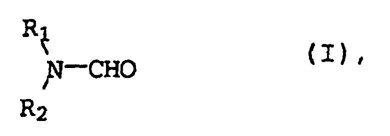 in der R¹ und R² unabhängig voneinander C₁₋ bis C₈-Alkyl oder R¹ und R² gemeinsam eine C₄- oder C₅-Alkylenkette, welche gegebenenfalls durch ein oder mehrere Sauerstoff- oder Stickstoff-Atome unterbrochen sein kann, bedeuten, insbesondere N,N-Dimethylformamid, N,N-Diethylformamid, N,N-Dipropylformamid, N,N-Diisopropylformamid, N,N-Dibutylformamid, N,N-Di-sek.-butylformamid oder N,N-Diisobutylformamid.

Durch Umsetzung des eingesetzten N,N-disubstituierten Formamids mit dem Chlorierungsmittel bildet sich das sogenannte Vilsmeier-Addukt als eigentlich reaktives Chlorierungsreagenz.

Bei der Herstellung von 3-Chlorpropin ist es ferner aus sicherheitstechnischen Gründen von Vorteil, die Bildung von Propargylchlorformiat durch Sicherstellung einer ausreichend hohen Katalysator- und Phosgen-Konzentration im Reaktionssystem zu unterbinden, da sich Propargylchlorformiat spontan unter Abspaltung von Kohlendioxid und Freisetzung einer erheblichen Energiemenge zersetzen kann.

In einer allgemeinen Ausführungsform zur eigensicheren Lagerung oder zum eigensicheren Transport von 3-Chlorpropin mischt man das 3-Chlorpropin mit einem geeigneten Verdünnungsmittel in einer Menge, die unter der gewünschten Temperatur und dem gewünschten Druck ausreicht, um die Flüssigphase und die Gasphase deflagrationsstabil zu halten. Gegebenenfalls ist zur Erfüllung der Druckbilanz zwischen dem gewünschten Gesamtdruck des Systems und der Summe der Partialdrücke von 3-Chlorpropin und dem Verdünnungsmittel noch eine weitere gasförmige Komponente zuzufügen.

In einer bevorzugten Ausführungsform zur eigensicheren Lagerung oder zum eigensicheren Transport von 3-Chlorpropin gibt man eine Mischung aus 15 bis 30 Gew.-% 3-Chlorpropin und 85 bis 70 Gew.-% Toluol in einen Behälter, welcher zuvor mit Inertgas, bevorzugt Stickstoff, gefüllt wurde. Dieser, im Allgemeinen verschlossene Behälter mit einem bevorzugten Anfangsinnendruck von etwa einer Atmosphäre bei der Abfülltemperatur kann nun bei den üblichen Temperaturen, bevorzugt 0 bis 50°C, gelagert und/oder transportiert werden, ohne daß sich darin eine deflagrationsfähige Flüssigphase oder eine deflagrationsfähige Gasphase bilden kann.

In einer allgemeinen Ausführungsform zur diskontinuierlichen Herstellung von 3-Chlorpropin gibt man die gewünschte Gesamtmenge an Propin-3-ol zusammen mit dem geeigneten Verdünnungsmittel in entsprechender Menge und den gewünschten Katalysator in einen Reaktionsbehälter. Anschließend beginnt man, das Gemisch auf die gewünschte Reaktionstemperatur aufzuheizen und die zur Umsetzung des Propin-3-ols erforderliche Menge an Chlorierungsmittel, bevorzugt Phosgen, unter intensiver Durchmischung einzuleiten. Nach Beendigung der Einleitung des Chlorierungsmittels läßt man gegebenenfalls noch einige Minuten bis wenige Stunden nachreagieren.

In einer bevorzugten Ausführungsform zur kontinuierlichen Herstellung von 3-Chlorpropin gibt man zum Anfahren der Reaktion eine kleine Menge an Propin-3-ol zusammen mit dem geeigneten Verdünnungsmittel in entsprechender Menge und den gewünschten Katalysator in einen Reaktionsbehälter. Anschließend beginnt man, das Gemisch auf die gewünschte Reaktionstemperatur aufzuheizen und die zur Umsetzung des Propin-3-ols erforderliche Menge an Phosgen unter intensiver Durchmischung einzuleiten. Alternativ kann man auch eine kleine Menge an 3-Chlorpropin zusammen mit dem geeigneten Verdünnungsmittel in entsprechender Menge und den gewünschten Katalysator in einen Reaktionsbehälter vorlegen, auf die gewünschte Reaktionstemperatur aufheizen und mit der Zugabe von Phosgen beginnen. Anschließend führt man nun kontinuierlich die Einsatzlösung, welche Propin-3-ol, Verdünnungsmittel und Katalysator enthält, und parallel dazu die erforderliche Menge an Phosgen hinzu. Nachdem eine bestimmte Menge an Einsatzlösung zugeführt, beziehungsweise ein bestimmter Flüssigkeitsstand im Reaktionsbehälter erreicht wurde, wird die Zufuhr an Phosgen beendet. Die Einsatzlösung wird in der Regel noch solange zugefahren, bis der Abgasstrom an gebildetem Chlorwasserstoff und Kohlendioxid versiegt, das heißt das in Lösung befindliche Phosgen umgesetzt wurde. Die erhaltene Rohlösung wird nun abgekühlt und in einen Lagerbehälter gefahren.

Des weiteren wurde die Verwendung von 3-Chlorpropin, welches gemäß dem erfindungsgemäßen Verfahren hergestellt, gelagert und/oder transportiert wurde, in der Synthese von Farbstoffen, Pharma- und Agrarwirkstoffen, Galvanohilfsmitteln, Desinfektionsmitteln, Steroiden und Wuchshormonen gefunden.

Das erfindungsgemäße Verfahren ermöglicht den eigensicheren Umgang mit 3-Chlorpropin durch Sicherstellung einer deflagrationsstabilen Flüssigphase und einer deflagrationsstabilen Gasphase durch die Gegenwart eines Verdünnungsmittels. Dabei wird die Deflagrationsstabilität beider Phasen durch die Art und Menge des Verdünnungsmittels sowie die Temperatur und den Gesamtdruck des Systems sichergestellt. Aufgrund dieser Sicherstellung, welche bereits durch die Einstellung des thermodynamischen Gleichgewichts zwischen beiden Phasen erreicht wird, ist es an keiner räumlichen Stelle des Systems möglich, eine Konzentration an 3-Chlorpropin zu erhalten, welche oberhalb der deflagrationsfähigen Konzentration liegt.

Durch das erfindungsgemäße Verfahren wird insbesondere die Lagerung, der Transport und die Herstellung von 3-Chlorpropin auf einem sehr hohen sicherheitstechnischen Stand ermöglicht.

### Beispiele

### Versuchsdurchführung 1: Bestimmung der Gasphasenkonzentration von 3-Chlorpropin in Abhängigkeit von der Flüssigphasenkonzentration

Die Versuche zur Bestimmung der Gasphasenkonzentration von 3-Chlorpropin in Abhängigkeit von der Flüssigphasenkonzentration wurden in einer Umlaufapparatur zur dynamischen Bestimmung von Gas-Flüssig-Phasengleichgewichtsdaten durchgeführt. Es wurden flüssige Mischungen von 3-Chlorpropin und einem Verdünnungsmittel sowie gegebenenfalls N,N-Diisobutylformamid (DIBF) vorgelegt und das Gas-Flüssig-Phasengleichgewicht eingestellt, wobei bei einer Vorgabe der Temperatur der sich einstellende Dampfdruck und bei Vorgabe des Drucks die sich einstellende Siedetemperatur gemessen wurde. Zur gaschromatographischen Analyse wurden Proben aus der Flüssigphase sowie aus der zuvor kondensierten Gasphase entnommen. Aus den gaschromatographischen Analysen wurden die Partialdrücke von 3-Chlorpropin und des Verdünnungsmittels berechnet.

### Versuchsdurchführung 2: Bestimmung der Deflagrationsgrenze von 3-Chlorpropin enthaltenden Gasmischungen

Die Versuche zur Bestimmung der Deflagrationsgrenze von Gasmischungen, das heißt der Grenzlinie zwischen den Zusammensetzungen der Gasmischungen, bei der auf der einen Seite gerade ein sich selbständig fortschreitender Zerfalls ausgelöst werden kann und auf der anderen Seite gerade kein sich selbständig fortschreitender Zerfalls ausgelöst werden kann, wurden in Anlehnung an die künftige europäische Norm prEN1839, Method B "bomb method", in einem kugelförmigen (sphärischen) und beheizbaren 5 L-Hochdruckbehälter durchgeführt (Arbeitsdokument der Physikalisch Technischen Bundesanstalt Braunschweig, Titel "CEN/TC 305/WG1/SG 4", Stand Januar 2000).

Die Gasmischungen wurden nach der Partialdruckmethode hergestellt. Dabei gilt unter der Annahme idealen Verhaltens, daß die mit Zugabe jeder Komponente herbeigeführte Druckerhöhung im Behälter dem Partialdruck dieser Komponente im Gesamtgemisch entspricht und daß der Anteil des Partialdrucks einer Komponente am Gesamtdruck identisch mit ihrem Volumenanteil und ihrem Molzahlanteil ist.

Zur guten Durchmischung wurden die Gase ca. 5 Minuten mit einem Magnetrilhrwerk mit Propeller durchmischt. Gezündet wurde mit einem schmelzenden Draht, bei dem nach dem Schmelzen ein Lichtbogen auftritt (siehe künftige europäische Norm prEN1839, Method B "bomb method", "fusing wire"). Die freigesetzte Zündenergie betrug ca. 70 J. Die durch die Zündung eventuell ausgelöste selbständige Ausbreitung einer Reaktionsfront (Deflagration) wurde über den zeitlichen Anstieg des Behälterinnendruckes über eine piezoelektrische Messung detektiert. Als Kriterium für eine Deflagration wurde der maximale, nach der Zündung im Behälter gemessene absolute Druck herangezogen. Betrug dieser mehr als das 1,05-fache des Anfangsdrucks vor der Zündung, so wurde das Gasgemisch als deflagrationsfähig eingestuft. Lag dieser beim 1,05-fachen Wert des Anfangsdrucks vor der Zündung oder darunter, so wurde das Gasgemisch als nicht-deflagrationsfähig eingestuft.

### Beispiel 1: Bestimmung der Gasphasenkonzentration und des Dampfdrucks von 3-Chlorpropin in Abhängigkeit von der Flüssigphasenkonzentration unter Einsatz von Toluol als Verdünnungsmittel

Die Messungen wurden nach der oben beschriebenen Versuchsdurchführung 1 durchgeführt.

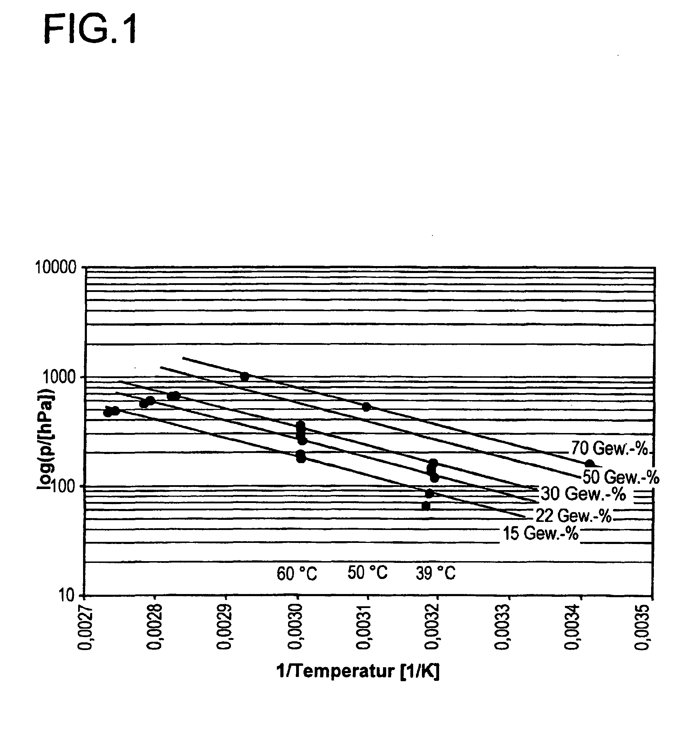

Unter Einsatz von Toluol als Verdünnungsmittel wurden vier Meßreihen mit unterschiedlichen Ausgangskonzentrationen von 3-Chlorpropin von etwa 15 Gew.-%, etwa 22 Gew.-%, etwa 30 Gew.-% und etwa 70 Gew.-% aufgenommen. Bei den ersten drei Messreihen war zudem N,N-Diisobutylformamid (DIBF) zugegen. Die Ergebnisse sind in den Tabellen 1a bis 1d dargestellt. Abbildung 1 zeigt eine grafische Auftragung des Dampfdrucks [log(p/hpa])] von 3-Chlorpropin für die genannten Ausgangskonzentrationen gegen die Temperatur [1/T] sowie die interpolierte Kurve für eine etwa 50 Gew.-% 3-Chlorpropin enthaltende Mischung.

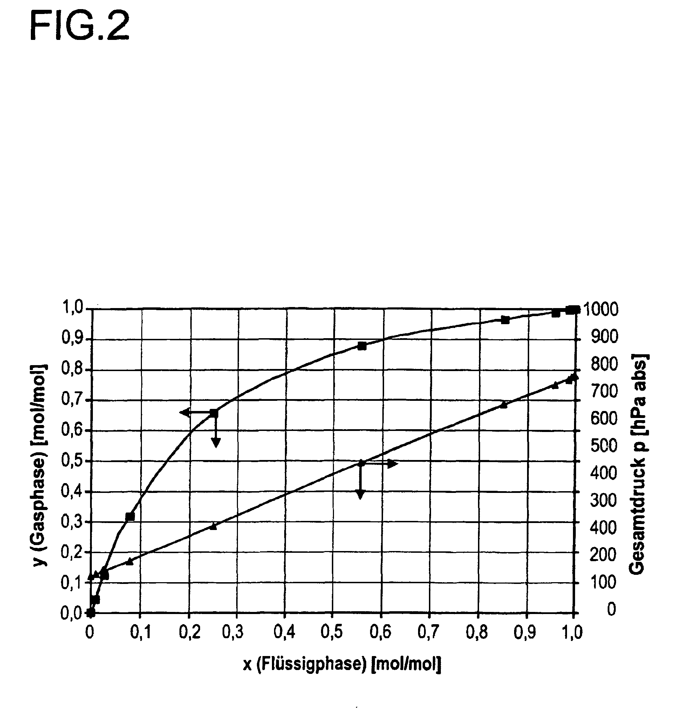

In einer fünften Meßreihe wurden Mischungen von 3-Chlorpropin und Toluol unterschiedlicher Ausgangskonzentrationen hergestellt, nach der oben beschriebenen Versuchsdurchführung 1 das Phasengleichgewicht bei einer konstanten Temperatur von 50°C eingestellt und die Gleichgewichts-Stoffmengenanteil von 3-Chlorpropin sowie der Gesamtdruck des Systems bestimmt. Die Ergebnisse sind in Tabelle 2 dargestellt. Abbildung 2 zeigt eine grafische Auftragung des Gleichgewichts-Stoffmengenanteils von 3-Chlorpropin in der Gasphase sowie des Gesamtdrucks des Systems in Abhängigkeit vom Gleichgewichts-Stoffmengenanteil von 3-Chlorpropin in der Flüssigphase.

**Tabelle 2: Gas-Flüssig-Phasengleichgewicht des Systems 3-Chlorpropin/Toluol bei 50°C**

| Druck p [hPa abs] | Gleichgewichts-Stoffmengenanteil von 3-Chlorpropin | |
|---|---|---|
| | x (in der Flüssigphase) | y (in der Gasphase) |
| 123 | 0 | 0 |
| 128 | 0,010 | 0,045 |
| 138 | 0,026 | 0,125 |
| 171 | 0,078 | 0,320 |
| 285 | 0,249 | 0,659 |
| 494 | 0,557 | 0,879 |
| 688 | 0,853 | 0,967 |
| 752 | 0,960 | 0,991 |
| 769 | 0,988 | 0,997 |
| 775 | 0,995 | 0,999 |
| 780 | 1 | 1 |

### Beispiel 2: Bestimmung der Gasphasenkonzentration und des Dampfdrucks von 3-Chlorpropin in Abhängigkeit von der Flüssigphasenkonzentration unter Einsatz von n-Hexan als Verdünnungsmittel

Die Messungen wurden nach der oben beschriebenen Versuchsdurchführung 1 durchgeführt. Unter Einsatz von n-Hexan als Verdünnungsmittel wurden Mischungen mit 3-Chlorpropin unterschiedlicher Ausgangskonzentrationen hergestellt, nach der oben beschriebenen Versuchsdurchführung 1 das Phasengleichgewicht bei einer konstanten Temperatur von 50°C eingestellt und der Gleichgewichts-Stoffmengenanteil von 3-Chlorpropin sowie der Gesamtdruck des Systems bestimmt. Die Ergebnisse sind in Tabelle 3 dargestellt.

**Tabelle 3: Gas-Flüssig-Phasengleichgewicht des Systems 3-Chlorpropin/n-Hexan bei 50°C**

| Druck p [hPa abs] | Gleichgewichts-Stoffmengenanteil von 3-Chlorpropin | |
|---|---|---|
| | x (in der Flüssigphase) | y (in der Gasphase) |
| 541 | 0 | 0 |
| 576 | 0,021 | 0,079 |
| 662 | 0,079 | 0,231 |
| 838 | 0,280 | 0,481 |
| 930 | 0,614 | 0,610 |
| 926 | 0,783 | 0,714 |
| 899 | 0,883 | 0,803 |
| 832 | 0,971 | 0,931 |
| 796 | 0, 994 | 0, 985 |
| 780 | 1 | 1 |

### Beispiel 3: Bestimmung der Gasphasenkonzentration und des Dampfdrucks von 3-Chlorpropin in Abhängigkeit von der Flüssigphasenkonzentration unter Einsatz von Cyclohexan als Verdünnungsmittel

Die Messungen wurden nach der oben beschriebenen Versuchsdurchführung 1 durchgeführt. Unter Einsatz von Cyclohexan als Verdünnungsmittel wurden Mischungen mit 3-Chlorpropin unterschiedlicher Ausgangskonzentrationen hergestellt, nach der oben beschriebenen Versuchsdurchführung 1 das Phasengleichgewicht bei einer konstanten Temperatur von 50°C eingestellt und die Gleichgewichts-Stoffmengenanteil von 3-Chlorpropin sowie der Gesamtdruck des Systems bestimmt. Die Ergebnisse sind in Tabelle 4 dargestellt.

**Tabelle 4: Gas-Flüssig-Phasengleichgewicht des Systems 3-Chlorpropin/Cyclohexan bei 50°C**

| Druck p [hPa abs] | Gleichgewichts-Stoffmengenanteil von 3-Chlorpropin | |
|---|---|---|
| | x (in der Flüssigphase) | y (in der Gasphase) |
| 363 | 0 | 0 |
| 389 | 0,015 | 0,082 |
| 469 | 0,063 | 0,266 |
| 673 | 0,256 | 0,561 |
| 782 | 0,527 | 0,691 |
| 822 | 0,759 | 0,787 |
| 805 | 0,967 | 0,953 |
| 790 | 0,993 | 0,989 |
| 780 | 1 | 1 |

### Beispiel 4: Bestimmung der Deflagrationsgrenze binärer Gasmischungen mit 3-Chlorpropin

Die Messungen wurden nach der oben beschriebenen Versuchsdurchführung 2 durchgeführt. Als zweite Gaskomponente wurden die Kohlenwasserstoffe Toluol, Cyclohexan beziehungsweise n-Hexan eingesetzt. Die ermittelten nicht-deflagrationsfähigen Bereiche sind in den Tabellen 5a bis 5c wiedergegeben. Die Messungen zeigen, daß auch die Art des Verdünnungsmittels einen entscheidenden Einfluß auf die Lage der Deflagrationsgrenze besitzt.

**Tabelle 5a: Gasgemisch aus 3-Chlorpropin und Toluol**

| | | |
|---|---|---|
| Nicht-deflagrationsfähig | 3-Chlorpropin | Toluol |
| in Gasphase bei | ≤ 71 Vol.-% | ≥ 29 Vol.-% |
| 100°C und 0,1 MPa abs | ≤ 66,5 Gew.-% | ≥ 33,5 Gew.-% |

**Tabelle 5b: Gasgemisch aus 3-Chlorpropin und Cyclohexan**

| | | |
|---|---|---|
| Nicht-deflagrationsfähig | 3-Chlorpropin | Cyclohexan |
| in Gasphase bei | ≤ 83 Vol.-% | ≥ 17 Vol.-% |
| 100°C und 0,1 MPa abs | ≤ 81,2 Gew.-% | ≥18,8 Gew.-% |

**Tabelle 5c: Gasgemisch aus 3-Chlorpropin und n-Hexan**

| | | |
|---|---|---|
| Nicht-deflagrationsfähig | 3-Chlorpropin | n-Hexan |
| in Gasphase bei | ≤ 80 Vol.-% | ≥ 20 Vol.-% |
| 80 bis 150°C und 0,1 MPa abs | ≤ 77,6 Gew.-% | ≥ 22,4 Gew.-% |

### Beispiel 5: Bestimmung der Deflagrationsgrenze ternärer Gasmischungen mit 3-Chlorpropin

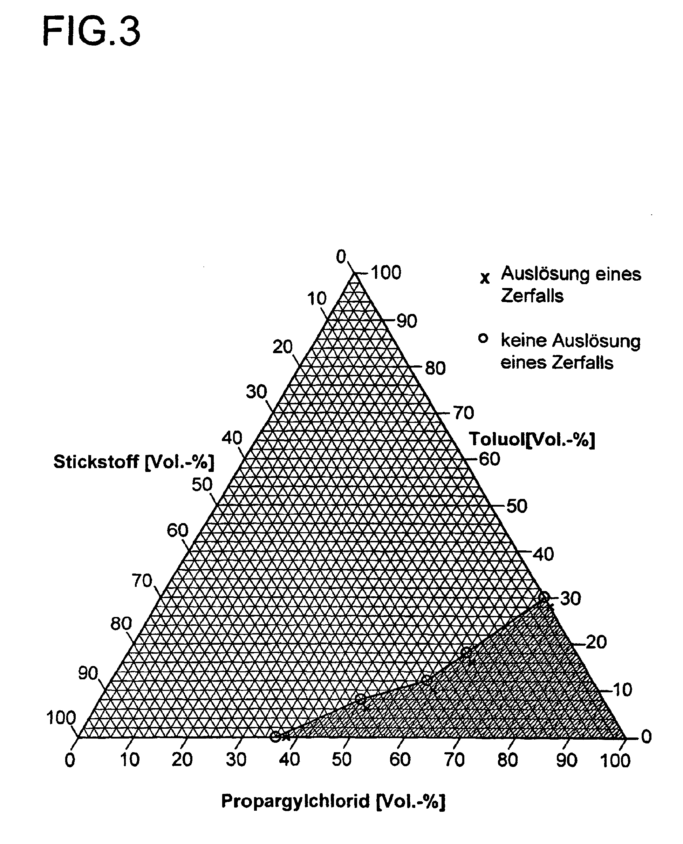

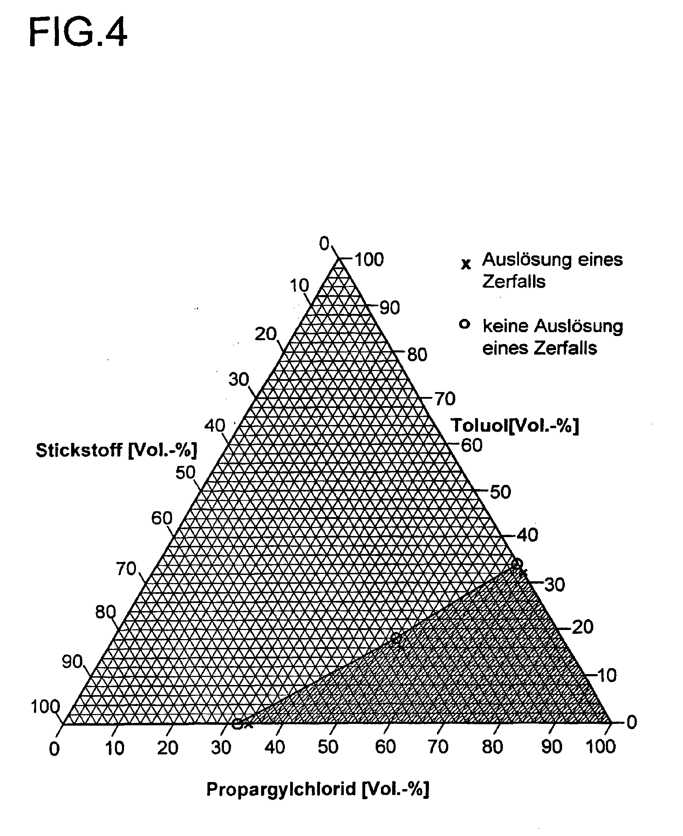

Die Messungen wurden nach der oben beschriebenen Versuchsdurchführung 2 durchgeführt. Als weitere Gaskomponenten wurden Toluol und Stickstoff eingesetzt. Die ermittelten Gaszusammensetzungen, bei denen gerade eine Deflagration beziehungsweise gerade keine Deflagration ausgelöst werden kann sind in den Tabellen 6a (für 0,10 MPa abs) und 6b (für 0,19 MPa abs) wiedergegeben. Die Deflagrationsdiagramne sind in den Abbildungen 3a und 3b dargestellt. Innerhalb der schraffierten Bereiche ist das Gasgemisch deflagrationsfähig. Die Messungen belegen, daß die Deflagrationsgrenze druckabhängig ist.

**Tabelle 6a: Gasgemisch aus 3-Chlorpropin, Toluol und Stickstoff bei 0,10 MPa abs**

| | T [°C] | 3-Chlorpropin [vol.-%] | Toluol [Vol.-%] | Stickstoff [Vol.-%] |
|---|---|---|---|---|
| Gaszusammen-setzungen, bei denen gerade eine Deflagration ausgelöst werden kann | 100 | 38 | 0 | 62 |
| | 100 | 50 | 6 | 44 |
| | 100 | 60 | 10 | 30 |
| | 100 | 64 | 16 | 20 |
| | 100 | 72 | 28 | 0 |
| Gaszusammen-setzungen, bei denen gerade keine Deflagration ausgelöst werden kann | 100 | 36 | 0 | 64 |
| | 100 | 48 | 8 | 44 |
| | 100 | 58 | 12 | 30 |
| | 100 | 62 | 18 | 20 |
| | 100 | 70 | 30 | 0 |

**Tabelle 6b: Gasgemisch aus 3-Chlorpropin, Toluol und Stickstoff bei 0,19 MPa abs**

| | T [°C] | 3-Chlorpropin [Vol.-%] | Toluol [Vol.-%] | Stickstoff [Vol.-%] |
|---|---|---|---|---|
| Gaszusammen-setzungen, bei denn gerade eine Deflagration ausgelöst werden kann | 100 | 34 | 0 | 66 |
| | 100 | 54 | 16 | 30 |
| | 120 | 68 | 32 | 0 |
| Gaszusammen-setzungen, bei denen gerade keine Deflagration ausgelöst werden kann | 100 | 32 | 0 | 68 |
| | 100 | 52 | 18 | 30 |
| | 120 | 66 | 34 | 0 |

### Beispiel 6: Halbkontinuierliche Herstellung von 3-Chlorpropin in Toluol als Verdünnungsmittel (erfindungsgemäß)

In eine Phosgenierungsapparatur, welche einen Mehrhalskolben mit Rührer und aufgesetzter Kühlkaskade (mit einem bei -10°C betriebenen Intensivkühler und aufgesetztem Kohlensäurekühler) umfasste, wurden 250 mL (226,6 g) einer vorab gemischten Lösung aus 112,2 g 3-Chlorpropin, 11,87 g N,N-Diisobutylformamid (DIBF) und 262 g Toluol vorgelegt. Die Lösung enthielt somit 29,1 Gew.-% 3-Chlorpropin und 67,9 Gew.-% Toluol. Die Vorlage wurde auf 50°C erwärmt und Phosgen eingegast. Nach Zugabe von etwa 65 g Phosgen setzte der Rückfluß über die Kühlkaskade ein. Mit Beginn des Phosgen-Rückflusses wurde mit der Dosierung von 447,3 g (500 mL) einer Lösung von 103,7 g Propin-3-ol und 14,6 g DIBF in 329 g Toluol begonnen. Die Lösung wurde innerhalb von 4 Stunden zugetropft. Parallel dazu wurden weitere 202 g Phosgen eingegast.

Während der Dosierung der Lösung und des Phosgens wurde aus der Gasphase des Mehrhalskolbens mittels eines evakuierten Glasgefäßes eine Gasprobe aus dem Gasraum der Reaktion genommen und bis zu einem Innendruck von 100 hPa abs befüllt, der Rest wurde bis zum Normaldruck durch Stickstoff aufgefüllt. Die gaschromatographische Analyse ergab eine Gasphasenzusammensetzung von ca. 29,5 Vol.-% Kohlendioxid, 0,2 Vol.-% Toluol, 18 Vol.-% 3-Chlorpropin, 19,7 Vol.-% Chlorwasserstoff und 32,6 Vol.-% Phosgen.

Nach dem Ende der Dosierung wurden innerhalb von 20 Minuten noch 3 g Propin-3-ol zugegeben, um das überschüssige Phosgen umzusetzen. Danach wurde die Reaktionsmischung zur Nachreaktion noch 1 Stunde bei 50°C belassen. Die gebildete Rohlösung enthielt laut gaschromatographischer Analyse ca. 27 Gew.-% 3-Chlorpropin, was einer Rohausbeute von etwa 93% entspricht.

Da nach D.R. Forshey et al. in Fire Technology 5 (1969) Seite 100 bis 111 bereits ein Zusatz von 10 Gew.-% an Toluol in der Flüssigphase ausreicht, um eine bis 4,3 MPa abs deflagrationsstabile Flüssigphase zu erhalten, ist auch im vorliegenden Beispiel die Flüssigphase deflagrationsstabil.

Die während der Umsetzung entnommene Gasprobe enthielt 18 Vol.-% 3-Chlorpropin und 0,2 Vol.-% Toluol. Wie aus dem Deflagrationsdiagramm in Abbildung 3a hervorgeht, ist ein ternäres Gasgemisch mit etwa 18 Vol.-% 3-Chlorpropin und etwa 0,2 Vol.-% Toluol selbst bei 100°C weit außerhalb des deflagrationsfähigen Bereichs.

Der Gehalt von 27 Gew.-% 3-Chlorpropin in der erhaltenen Rohlösung entspricht einem Stoffmengenanteil x in der Flüssigphase von etwa 0,31. Nach dem in Beispiel 1 ermittelten Gas-Flüssig-Phasengleichgewicht des Systems 3-Chlorpropin/Toluol bei 50°C ergibt sich anhand der Auftragung in Abbildung 2 bei 50°C ein Stoffmengenanteil von 3-Chlorpropin in der Gasphase von etwa 0,72 und ein Druck von 3-Chlorpropin und Toluol in Summe von etwa 330 hPa. Dies entspricht einem 3-Chlorpropin-Partialdruck von etwa 238 hPa und einem Toluol-Partialdruck von etwa 92 hPa. Da der Gesamtdruck im System etwa 0,1 MPa abs betrug, enthielt die Gasphase noch etwa 0,67 MPa an weiteren Gasen. Diese bestanden zum überwiegenden Teil aus den gebildeten Reaktions-Nebenprodukten Chlorwasserstoff und Kohlendioxid. Der 3-Chlorpropin-Partialdruck von etwa 238 hPa ist bei einer Gasmischung von einem Gesamtdruck von 0,1 MPa abs einem 3-Chlorpropin-Anteil von etwa 23,8 Vol.-% gleichzusetzen. Der vorhandene Toluol-Partialdruck entspricht einem Toluol-Anteil von etwa 9,2 Vol.-%.

Wie aus dem Deflagrationsdiagramm in Abbildung 3a hervorgeht, ist ein ternäres Gasgemisch mit etwa 23,8 Vol.-% 3-Chlorpropin und etwa 9,2 Vol.-% Toluol selbst bei 100°C weit außerhalb des deflagrationsfähigen Bereichs.

### Beispiel 7: Halbkontinuierliche Herstellung von 3-Chlorpropin in Toluol als Verdünnungsmittel (erfindungsgemäß)

In eine Phosgenierungsanlage, welche einen mantelbeheizten 8 m³ Rührkessel und eine Kühlkaskade (mit einer ersten Kühlstufe bei -20°C und einer zweiten Kühlstufe bei -70°C) umfasste, wurden bei einer Manteltemperatur von 30°C 400 L einer Einsatzlösung bestehend aus 83,75 Gew.-% Toluol, 15,5 Gew.-% Propin-3-ol und 0,75 Gew.-% N,N-Diisobutylformamid (DIBF) vorgelegt. Unter Rühren wurden 75 L flüssiges Phosgen zudosiert und dabei die Innentemperatur auf 48°C angehoben. Durch die Umsetzung des Phosgens mit Propin-3-ol wurden Kohlendioxid und Chlorwasserstoff gebildet, welche als Abgasstrom nach der Kühlkaskade ausgeschleust wurden. Nach dem Nachlassen des Abgasstroms wurden bei einer Manteltemperatur von 50°C aus einer Vorlage ein konstanter Mengenstrom von 750 kg/h der Einsatzlösung (Zusammensetzung wie oben beschrieben) sowie ein konstanter Strom an Phosgen zugeführt. Der Druck im Reaktor betrug etwa 0,1 MPa abs. Die Phosgen-Zulaufmenge wurde dabei derart eingestellt, daß die Reaktorinnentemperatur bei 48°C konstant blieb. Die bei der Umsetzung beobachtete Abgasmenge nach der Kühlkaskade betrug etwa 100 m³/h. Die Zusammensetzung der Gasphase vor Eintritt in die Kühlkaskade wurde gaschromatographisch analysiert. Sie enthielt 33,7 Vol.-% Kohlendioxid, 31,3 vol.-% Chlorwasserstoff, 15 Vol.-% Phosgen, 17 Vol.-% 3-Chlorpropin und 3 Vol.-% Toluol. Die Abgastemperatur vor Eintritt in die Kühlkaskade betrug 35°C.

Nach Erreichen einer Füllmenge im Rührkessel von 5,2 m³ wurde der Phosgenzulauf geschlossen. Bei 48°C wurde noch weitere Einsatzlösung (Zusammensetzung wie oben beschrieben) nachdosiert, um noch gelöstes Phosgen umzusetzen. Nach dem Absinken des Abgasstroms nach der Kühlkaskade auf 0 m³/h wurde auch die Nachdosierung der Einsatzlösung beendet und die Reaktionsmischung weitere 2 Stunden bei 50°C zur Nachreaktion belassen. Die gebildete Rohlösung enthielt laut gaschromatographischer Analyse ca. 19,7 Gew.-% 3-Chlorpropin, was einer Rohausbeute von etwa 99% entspricht. Der Restgehalt an Phosgen lag bei weniger als 100 Gew.-ppm.

Da nach D.R. Forshey et al. in Fire Technology 5 (1969) Seite 100 bis 111 bereits ein Zusatz von 10 Gew.-% an Toluol in der Flüssigphase ausreicht, um eine bis 4,3 MPa abs deflagrationsstabile Flüssigphase zu erhalten, ist auch im vorliegenden Beispiel die Flüssigphase deflagrationsstabil.

Der Gehalt von 19,7 Gew.-% 3-Chlorpropin der erhaltenen Rohlösung entspricht einem Stoffmengenanteil x in der Flüssigphase von etwa 0,21. Nach dem in Beispiel 1 ermittelten Gas-Flüssig-Phasengleichgewicht des Systems 3-Chlorpropin/Toluol bei 50°C ergibt sich anhand der Auftragung in Abbildung 2 bei 50°C ein Stoffmengenanteil von 3-Chlorpropin in der Gasphase von etwa 0,60 und ein Druck von 3-Chlorpropin und Toluol in Summe von etwa 260 hPa. Dies entspricht einem 3-Chlorpropin-Partialdruck von etwa 156 hPa und einem Toluol-Partialdruck von etwa 104 hPa. Da der Gesamtdruck im System etwa 0,1 MPa abs betrug, enthielt die Gasphase noch etwa 0,74 MPa an weiteren Gasen. Diese bestanden zum überwiegenden Teil aus den gebildeten Reaktions-Nebenprodukten Chlorwasserstoff und Kohlendioxid. Der 3-Chlorpropin-Partialdruck von etwa 156 hPa ist bei einer Gasmischung von einem Gesamtdruck von 0,1 MPa abs einem 3-Chlorpropin-Anteil von etwa 15,6 Vol.-% gleichzusetzen. Der vorhandene Toluol-Partialdruck entspricht einem Toluol-Anteil von etwa 10,4 Vol.-%.

Wie aus dem Deflagrationsdiagramm in Abbildung 3a hervorgeht, ist ein ternäres Gasgemisch mit etwa 15,6 Vol.-% 3-Chlorpropin und etwa 10,4 Vol.-% Toluol selbst bei 100°C weit außerhalb des deflagrationsfähigen Bereichs.

### Beispiel 8: Halbkontinuierliche Herstellung von 3-Chlorpropin in Toluol gemäß Beispiel 5 aus WO 99/46226 (Vergleichsbeispiel)

In Beispiel 5 der WO 99/46226 wurden 22 g N,N-Diisobutylformamid (DIBF) in 70 g Toluol vorgelegt und bei einer konstanten Temperatur von 50°C über 3,5 Stunden hinweg 112 g Propin-3-ol und 230 g Phosgen zugefahren. Nach einer Nachreaktionszeit von 1 Stunde bei 50°C wurde nochmals Propin-3-ol zugegeben, um überschüssiges Phosgen abzureagieren. Nach einer weiteren Nachreaktionszeit von 1 Stunde wurde der Reaktionsaustrag gaschromatographisch analysiert. Er enthielt 65 Gew.-% 3-Chlorpropin.

Da nach D.R. Forshey et al. in Fire Technology 5 (1969) Seite 100 bis 111 bereits ein Zusatz von 10 Gew.-% an Toluol in der Flüssigphase ausreicht, um eine bis 4,3 MPa abs deflagrationsstabile Flüssigphase zu erhalten, ist auch im vorliegenden Beispiel die Flüssigphase deflagrationsstabil.

Der Gehalt von 65 Gew.-% 3-Chlorpropin der erhaltenen Rohlösung entspricht einem Stoffmengenanteil x in der Flüssigphase von etwa 0,70. Nach dem in Beispiel 1 ermittelten Gas-Flüssig-Phasengleichgewicht des Systems 3-Chlorpropin/Toluol bei 50°C ergibt sich anhand der Auftragung in Abbildung 2 bei 50°C ein Stoffmengenanteil von 3-Chlorpropin in der Gasphase von etwa 0,94 und ein Druck von 3-Chlorpropin und Toluol in Summe von etwa 590 hPa. Dies entspricht einem 3-Chlorpropin-Partialdruck von etwa 555 hPa und einem Toluol-Partialdruck von etwa 35 hPa. Da der Gesamtdruck im System etwa 0,1 MPa abs betrug, enthielt die Gasphase noch etwa 0,41 MPa an weiteren Gasen. Diese bestanden zum überwiegenden Teil aus den gebildeten Reaktions-Nebenprodukten Chlorwasserstoff und Kohlendioxid. Der 3-Chlorpropin-Partialdruck von etwa **555** hPa ist bei einer Gasmischung von einem Gesamtdruck von 0,1 MPa abs einem 3-Chlorpropin-Anteil von etwa 55,5 Vol.-% gleichzusetzen. Der vorhandene Toluol-Partialdruck entspricht einem Toluol-Anteil von etwa 3,5 Vol.-%.

Wie aus dem Deflagrationsdiagramm in Abbildung 3a hervorgeht, ist ein ternäres Gasgemisch mit etwa 55,5 Vol.-% 3-Chlorpropin und etwa 3,5 Vol.-% Toluol bei 100°C weit innerhalb des deflagrationsfähigen Bereichs. Unter den in Beispiel 5 der WO 99/46226 beschriebenen Bedingungen besteht somit das große Sicherheitsrisiko einer unkontrollierbaren Deflagration.

### Beispiel 9: Halbkontinuierliche Herstellung von 3-Chlorpropin in

Cyclohexan als Verdünnungsmittel (erfindungsgemäß) 151 g einer Mischung aus 50 Gew.-% (75 g) 3-Chlorpropin, 5 Gew.-% (8 g) N,N-Diisobutylformamid (DIBF) und 45 Gew.-% (68 g) Cyclohexan wurden in einer Phosgenierungsapparatur, welche einen Mehrhalskolben mit Rührer und aufgesetzter Kühlkaskade (mit einem bei -10°C betriebenen Intensivkühler und aufgesetztem Kohlensäurekühler) umfasste, vorgelegt und auf 50°C aufgeheizt. Bei 50°C wurden innerhalb von 30 Minuten 25 g Phosgen bis zum einsetzenden Rückfluß gasförmig zudosiert. Aus zwei 250 mL-Tropftrichtern wurden dann parallel eine Mischung aus 112 g Propin-3-ol und 15,73 g DIBF sowie 99,34 g Cyclohexan unter gleichzeitigem Eingasen von weiteren 200 g Phosgen innerhalb von 3,5 Stunden bei einer Innentemperatur von etwa 50°C zugegeben.

Nach 1,5 Stunden Reaktionszeit wurde mittels eines evakuierten Glasgefäßes eine Gasprobe aus dem Gasraum der Reaktion genommen und bis zu einem Innendruck von 100 hPa abs befüllt, der Rest wurde bis zum Normaldruck durch Stickstoff aufgefüllt. Die gaschromatographische Analyse ergab eine Gasphasenzusanunensetzung von ca. 44 Vol.-% Kohlendioxid, 6 Vol.-% Cyclohexan, 18,5 Vol.-% 3-Chlorpropin, 19 Vol.-% Chlorwasserstoff und 4,5 Vol.-% Phosgen.

Nach dem Ende der Dosierung wurden noch 10 g Propin-3-ol zugegeben, um das überschüssige Phosgen umzusetzen. Danach wurde die Reaktionsmischung zur Nachreaktion noch 1,5 Stunden bei 50°C belassen. Die gebildete Rohlösung enthielt laut gaschromatographischer Analyse ca. 52 Gew.-% 3-Chlorpropin, 42 Gew.-% Cyclohexan und 6 Gew.-% DIBF. Dies entspricht einer Rohausbeute an 3-Chlorpropin von etwa 99%. Die Gasphasenzusammensetzung einer 50 Gew.-%igen Lösung von 3-Chlorpropin in Cyclohexan beträgt bei 50°C etwa 69 Vol.-% 3-Chlorpropin und etwa 31 Vol.-% Cyclohexan. Die Konzentration an 3-Chlorpropin liegt somit deutlich unterhalb der in Beispiel 4, Tabelle 5b ermittelten Deflagrations-Grenzkonzentration von 83 Vol.-%.

Gemäß der oben beschriebenen Versuchsdurchführung zur Bestimmung der Deflagrationsgrenze von Gasmischungen wurde versucht, eine Gasmischung mit vergleichbarer Zusammensetzung wie die oben genannte Gasprobe zur Zündung zu bringen. Das Gasgemisch war nicht zündfähig.

Der Versuch zeigt, daß durch die Zugabe von Cyclohexan, einem deutlich niedriger als Toluol siedenden Verdünnungsmittel, eine Konzentration an 3-Chlorpropin in der Flüssigphase von über 50 Gew.-% ermöglicht werden kann, ohne daß die Gasphase während oder nach dem Ende der Reaktion eine deflagrationsfähige Zusammensetzung aufweist.

### Beispiel 10: Halbkontinuierliche Herstellung von 3-Chlorpropin in Toluol/Pentan als Verdünnungsmittel (erfindungsgemäß)

In eine Phosgenierungsapparatur, welche einen Mehrhalskolben mit Rührer und aufgesetzter Kühlkaskade (mit einem bei -10°C betriebenen Intensivkühler und aufgesetztem Kohlensäurekühler) umfasste, wurde eine Lösung aus 54 g 3-Chlorpropin, 5,72 g N,N-Diisobutylformamid (DIBF), 102,32 g Toluol und 18 g n-Pentan/iso-Pentan-Gemisch (mit 95%-igem Anteil an n-Pentan, Fa. Haltermann) vorgelegt. Die Lösung enthielt somit 30 Gew.-% 3-Chlorpropin, 56,8 Gew.-% Toluol und 10 Gew.-% n-Pentan/isc-Pentan-Gemisch. Die Vorlage wurde auf 50°C erwärmt und 22 g Phosgen eingegast. Anschließend wurden parallel 540 g einer Einsatzlösung aus 131,68 g Propin-3-ol und 18,48 g DIBF in 331,56 g Toluol und 58,32 g n-Pentan/iso-Pentan-Gemisch sowie 240 g Phosgen bei einer Innentemperatur von 50 bis 51°C innerhalb von 4 Stunden zugegeben.

Während der Dosierung der Lösung und des Phosgens wurde aus der Gasphase des Mehrhalskolbens mittels eines evakuierten Glasgefäßes eine Gasprobe aus dem Gasraum der Reaktion genommen und bis zu einem Innendruck von 100 hPa abs befüllt, der Rest wurde bis zum Normaldruck durch Stickstoff aufgefüllt. Die gaschromatographische Analyse ergab eine Gasphasenzusammensetzung von ca. 23,3 Vol.-% Kohlendioxid, 0,1 Vol.-% Toluol, 18 Vol.-% 3-Chlorpropin, 18,7 Vol.-% Chlorwasserstoff, 33,6 Vol.-% Pentanisomere und 6,3 Vol.-% Phosgen.

Nach dem Ende der Dosierung wurden noch 7,86 g Propin-3-ol zugegeben, um das überschüssige Phosgen umzusetzen. Danach wurde die Reaktionsmischung zur Nachreaktion noch 2 Stunden bei 50°C belassen. Die gebildete Rohlösung enthielt laut gaschromatographischer Analyse ca. 30 Gew.-% 3-Chlorpropin, 56,4 Gew.-% Toluol, 9,6 Gew.-% Pentanisomere und 3,3 Gew.-% DIBF. Dies entspricht einer Rohausbeute an 3-Chlorpropin von etwa 99%. Die Gasphasenzusammensetzung über dem Reaktionsaustrag bei 50°C betrug ca. 48 Vol.-% 3-Chlorpropin, 25 Vol.-% Pentanisomere und 24 Vol.-% Toluol.

Da nach D.R. Forshey et al. in Fire Technology 5 (1969) Seite 100 bis 111 bereits ein Zusatz von 10 Gew.-% an Toluol in der Flüssigphase ausreicht, um eine bis 4,3 MPa abs deflagrationsstabile Flüssigphase zu erhalten, ist auch im vorliegenden Beispiel die Flüssigphase deflagrationsstabil.

Aus der Gasphasenzusammensetzung über dem Reaktionsaustrag ergibt sich ein 3-Chlorpropin-Stoffmengenanteil von etwa 0,48, ein Toluol-Stoffmengenanteil von etwa 0,24 und ein Pentanisomere-Stoffmengenanteil von etwa 0,25. Aus dem Deflagrationsdiagramm in Abbildung 3a geht hervor, daß selbst ein ternäres Gasgemisch mit einem 3-Chlorpropin-Stoffmengenanteil von 0,48, einem Toluol-Stoffmengenanteil von 0,24 und einem Stickstoff-Stoffmengenanteil von 0,28 deutlich im deflagrationsstabilen Bereich liegt. Da aufgrund der höheren Wärmekapazität der Pentanisomere gegenüber Stickstoff die deflagrationsmindernde Wirkung von Pentanisomeren höher ist als jene von Stickstoff, ist die Gasphase über dem Reaktionsaustrag noch weiter im deflagrationsstabilen Bereich als die betrachtete 3-Chlorpropin/Toluol/Stickstoff-Gasmischung.

### Beispiel 11: Halbkontinuierliche Herstellung von 3-Chlorpropin in Toluol/Methyl-tert.-butylether als Verdünnungsmittel (erfindungsgemäß)

In eine Phosgenierungsapparatur, welche einen Mehrhalskolben mit Rührer und aufgesetzter Kühlkaskade (mit einem bei -10°C betriebenen Intensivkühler und aufgesetztem Kohlensäurekühler) umfasste, wurde eine Lösung aus 54 g 3-Chlorpropin, 5,72 g N,N-Diisobutylformamid (DIBF), 84,32 g Toluol und 36 g Methyl-tert.-butylether (MTBE) vorgelegt. Die Lösung enthielt somit 30 Gew.-% 3-Chlorpropin, 46,8 Gew.-% Toluol und 20 Gew.-% MTBE. Die Vorlage wurde auf 50°C erwärmt und 22 g Phosgen eingegast. Anschließend wurden parallel 540 g einer Einsatzlösung aus 131,68 g Propin-3-ol und 18,48 g DIBF in 273,24 g Toluol und 116,64 g MTBE sowie 234 g Phosgen bei einer Innentemperatur von 50 bis 52°C innerhalb von 4 Stunden zugegeben.

Während der Dosierung der Lösung und des Phosgens wurde aus der Gasphase des Mehrhalskolbens mittels eines evakuierten Glasgefäßes eine Gasprobe aus dem Gasraum der Reaktion genommen und bis zu einem Innendruck von 100 hPa abs befüllt, der Rest wurde bis zum Normaldruck durch Stickstoff aufgefüllt. Die gaschromatographische Analyse ergab eine Gasphasenzusammensetzung von ca. 26 Vol.-% Kohlendioxid, 0,03 Vol.-% Toluol, 10,4 Vol.-% 3-Chlorpropin, 31,1 Vol.-% Chlorwasserstoff, 2,6 Vol.-% MTBE und 29,8 Vol.-% Phosgen.

Nach dem Ende der Dosierung wurden noch 3,14 g Propin-3-ol zugegeben, um das überschüssige Phosgen umzusetzen. Danach wurde die Reaktionsmischung zur Nachreaktion noch 0,5 Stunden bei 50°C belassen. Die gebildete Rohlösung enthielt laut gaschromatographischer Analyse ca. 33 Gew.-% 3-Chlorpropin, 45,5 Gew.-% Toluol, 15,6 Gew.-% MTBE und 3,2 Gew.-% DIBF. Dies entspricht einer Rohausbeute an 3-Chlorpropin von etwa 99%. Die Gasphasenzusammensetzung über dem Reaktionsaustrag bei 50°C betrug ca. 53 Vol.-% 3-Chlorpropin, 24 Vol.-% MTBE und 15 Vol.-% Toluol.

Da nach D.R. Forshey et al. in Fire Technology 5 (1969) Seite 100 bis 111 bereits ein Zusatz von 10 Gew.-% an Toluol in der Flüssigphase ausreicht, um eine bis 4,3 MPa abs deflagrationsstabile Flüssigphase zu erhalten, ist auch im vorliegenden Beispiel die Flüssigphase deflagrationsstabil.

Aus der Gasphasenzusammensetzung über dem Reaktionsaustrag ergibt sich ein 3-Chlorpropin-Stoffmengenanteil von etwa 0,53, ein Toluol-Stoffmengenanteil von etwa 0,15 und ein MTBE-Stoffmengenanteil von etwa 0,24. Aus dem Deflagrationsdiagramm in Abbildung 3a geht hervor, daß selbst ein ternäres Gasgemisch mit einem 3-Chlorpropin-Stoffmengenanteil von 0,53, einem Toluol-Stoffmengenanteil von 0,15 und einem Stickstoff-Stoffmengenanteil von 0,32 deutlich im deflagrationsstabilen Bereich liegt. Da aufgrund der höheren Wärmekapazität von MTBE gegenüber Stickstoff die deflagrationsmindernde Wirkung von MTBE höher ist als jene von Stickstoff, ist die Gasphase über dem Reaktionsaustrag noch weiter im deflagrationsstabilen Bereich als die betrachtete 3-Chlorpropin/Toluol/Stickstoff-Gasmischung.

### Beispiel 12: Untersuchung der Deflagrationsfähigkeit während und nach der Synthese von 3-Chlorpropin unter Einsatz von Toluol als Verdünnungsmittel im Bereich von 25 bis 45 Gew.-% 3-Chlorpropin

Unter den in Beispiel 6 beschriebenen Versuchsbedingungen wurden in einem 4 L-Reaktionskolben, in dem zusätzlich zum beschriebenen Aufbau noch eine Hochspannungsfunkenstrecke installiert war, durch entsprechende Anpassung des Verhältnisses von Propin-3-ol und Toluol, Lösungen mit einem Endgehalt an 3-Chlorpropin von 25 Gew.-%, 30 Gew.-%, 35 Gew.-%, 40 Gew.-% und 45 Gew.-% hergestellt. Bei diesen Synthesen wurde mit Beginn des Phosgenrückflusses während der gesamten Reaktionsdauer bis zum Versuchsende im 10-Minutenabstand ein Zündfunke mit einer Energie von ca. 20 J eingetragen.

Bei allen Versuchen konnte keine Deflagration ausgelöst werden.

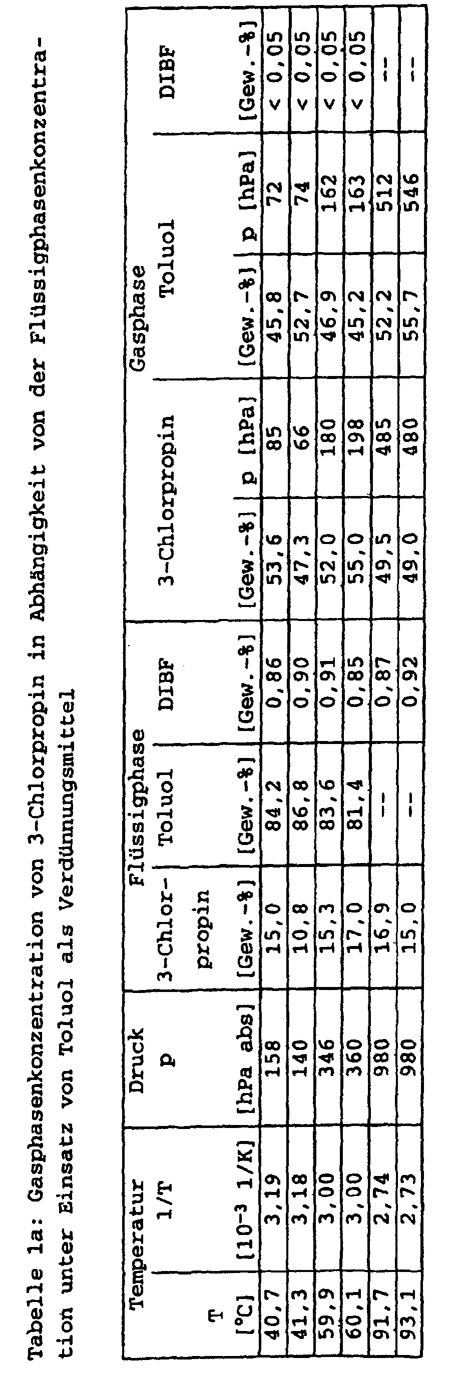

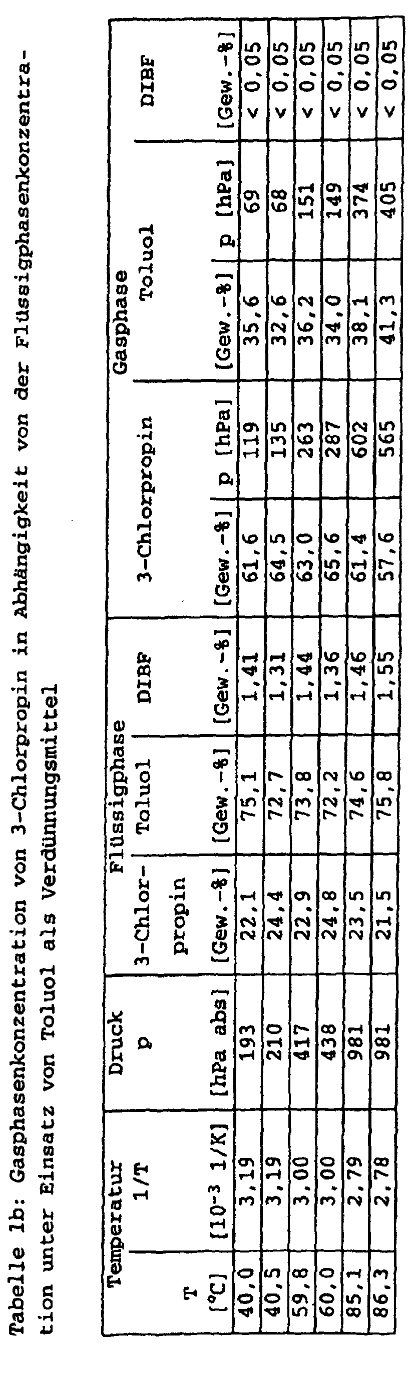

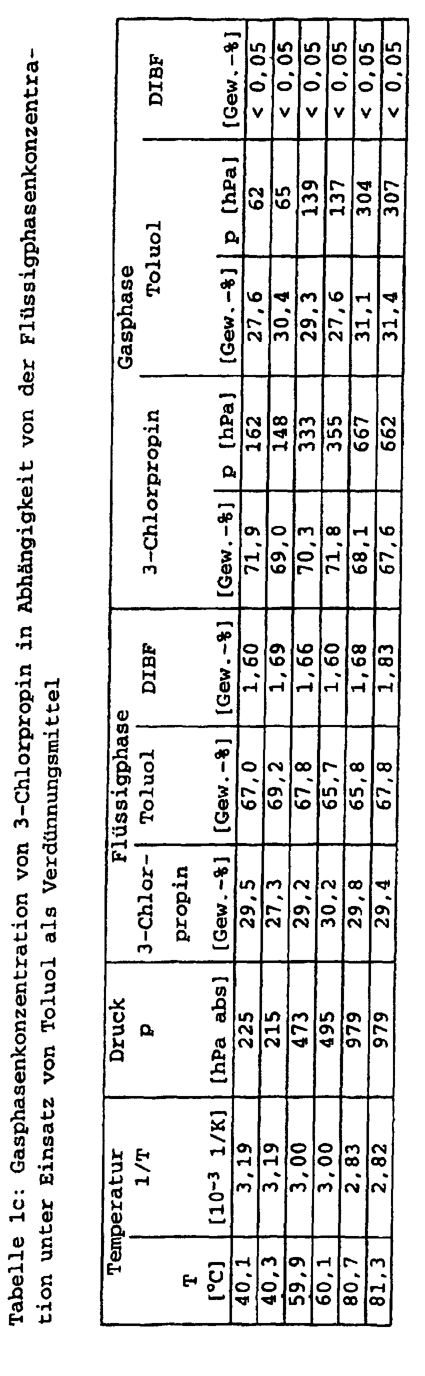

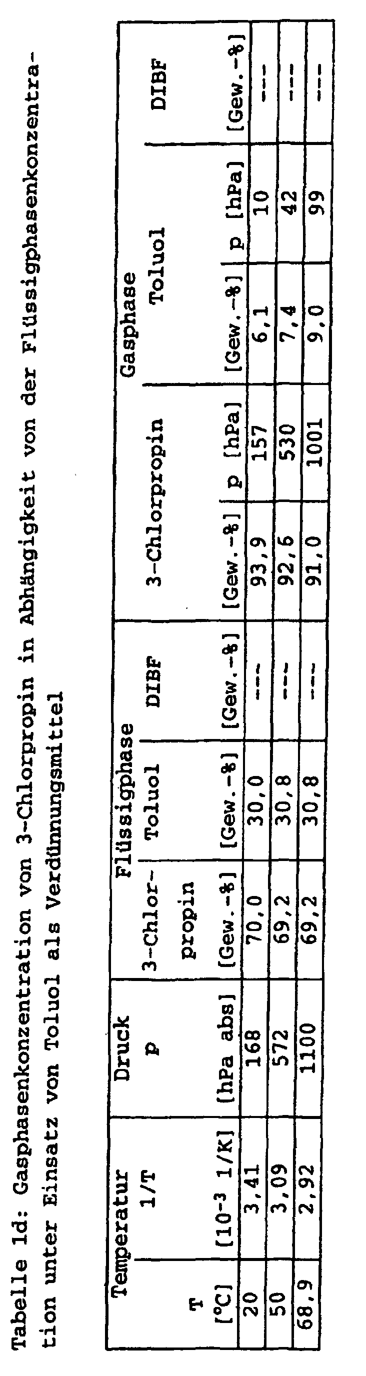

## Patentansprüche

1. Verfahren zum eigeneicheren Umgang mit 3-Ghlorpropin in Gegenwart eines Verdünnungsmittels mit einem Siedepunkt unter Normaldruck im Bereich von -50°C (223 K) bis 200°C (473 K)*,* **dadurch gekennzeichnet, daß** man ein gegenüber 3-Chlorpropin gleich oder höher siedendes Verdünnungsmittel in einer Menge von 50 bis 2000 Gew.-% in der Flüssigphase, bezogen auf die Menge an 3-chlorpropin in der Flüssigphase, einsetzt und man die Konzentration an 3-Chlorpropin in der Flüssigphase und in der Gasphase durch Art und Menge des Verdünnungsmittels, durch die Temperatur und durch den Gesamtdruck des Systems unterhalb der deflagrationsfähigen Konzentrationen hält.

2. Verfahren nach Anspruch 1, **dadurch gekennzeichnet, daß** man das gegenüber 3-Chlorpropin gleich oder höher siedende verdünnungsmittel in einer Menge von 50 bis 1000 Cew.-% in der Flüssigphase, bezogen auf die Menge an 3-Chlorpropin in der Flüssigphase, einsetzt.

3. Verfahren nach den Ansprüchen 1 bis 2, **dadurch gekennzeichnet, daß** man als gegenüber 3-Chlorpropin gleich oder hoher siedendes Verdünnungsmittel ein unverzweigtes oder verzweigtes C₆- bis C₈-Alkan, ein unverzweigtes oder verzweigtes C₆₋bis C₈-Cycloalkan mit einem 5- oder 6- Ring, Benzol, Toluol, Ethylbenzol, Xylole, oder Gemische davon einsetzt.

4. Verfahren nach Anspruch 1, **dadurch gekennzeichnet, daß** man eine Mischung aus (a) einem gegenüber 3-Chlorpropin gleich oder höher siedenden Verdünnungsmittel und (b) einem gegenüber 3-Chlorpropin niedriger siedenden Verdünnungsmittel einsetzt.

5. Verfahren nach Anspruch 4, **dadurch gekennzeichnet, daß** man (a) als gegenüber 3-Chlorpropin gleich oder höher siedendes Verdünnungsmittel ein unverzweigtes oder verzweigtes C₆- bis C₈-Alkan, ein unverzweigtes oder verzweigtes C₆- bis C₈-cycloalkan mit einem 5- oder 6- Ring, Benzol, Toluol, Ethylbenzol. Xylole, oder Gemische davon, und (b) als gegenüber 3-Chlorpropin niedriger siedendes Verdünnungsmittel ein C₃- bis C₅-Alkan, Cyclopentan, einen aliphatischen Ether mit insgesamt 2 bis 5 Kohlenstoffatomen, Chlormethan, oder Gemische davon einsetzt.

6. Verfahren nach den Ansprüchen 1 bis 5, **dadurch gekennzeichnet, daß** man die Druckbilanz zwischen dem Gesamtdruck des Systems und dem partialdruck von 3-Chlorpropin sowie dem partialdruck des Verdünnungsmittels durch Gegenwart eines Inertgases ausgleicht.

7. Verfahren nach den Ansprüchen 1 bis 6, **dadurch gekennzeichnet, daß** man das System bei einer Temperatur im Bereich von 0 bis 100°C und bei einem Gesamtdruck im Bereich 0,05 bis 0,5 MPa abs hält.

8. Verfahren nach den Ansprüchen 1 bis 7, **dadurch gekennzeichnet, daß** man das 3-Chlorpropin lagert oder transportiert.

9. Verfahren nach den Ansprüchen 1 bis 7, **dadurch gekennzeichnet, daß** man das 3-Chlorpropin durch Umsetzung von Propin-3-ol mit einem Chlorierungsmittel in Gegenwart eines Katalysators herstellt.

10. verfahren nach Anspruch 9, **dadurch gekennzeichnet, daß** man als Chlorierungsmittel Phosgen und als Katalysator ein N,N-disubstituiertes Formamid der allgemeinen Formel (I) 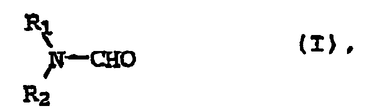 in der R¹ und R² unabhängig voneinander C₁- bis C₈-Alkyl oder R¹ und R² gemeinsam eine C₄- oder C₅-Alkylenkette, welche gegebenenfalls durch ein oder mehrere Sauerstoff- oder Stickstoff-Atome unterbrochen sein kann, bedeuten, einsetzt.

11. Verwendung von 3-Chlorpropin , welches gemäß der Ansprüche 1 bis 7 und 9 bis 10 hergestellt und/oder gemäß der Ansprüche 1 bis 8 gelagert und/oder transportiert wurde, in der Synthese von Farbstoffen, Pharma- und Agraxwirkstoffen, Galvanohilfsmitteln, Desinfektionsmitteln, Steroiden und Wuchshormonen.

## Claims

1. A method for the intrinsically safe handling of 3-chloropropyne in the presence of a diluent with a boiling point ranging from -50°C (223 K) to 200°C (473 K) under atmospheric pressure, wherein the diluent boiling at the same temperature as 3-chloropropyne or above is used in an amount of 50 to 2000% by weight in the liquid phase, based on the amount of 3-chloropropyne in the liquid phase, and the concentration of 3-chloropropyne in the liquid phase and in the gas phase is kept below the concentrations capable of deflagration by means of the type and amount of the diluent, the temperature and the total system pressure.

2. The method according to claim 1 wherein the diluent boiling at the same temperature as 3-chloropropyne or above is used in an amount of 50 to 1000% by weight in the liquid phase, based on the amount of 3-chloropropyne in the liquid phase.

3. The method according to claim 1 or 2 wherein the diluent used, which boils at the same temperature as 3-chloropropyne or above, is an unbranched or branched C₆- to C₈-alkane, an unbranched or branched C₆- to C₈-cycloalkane with a 5-membered or 6-membered ring, benzene, toluene, ethylbenzene, xylenes or mixtures thereof.

4. The method according to claim 1 wherein a mixture of (a) a diluent boiling at the same temperature as 3-chloropropyne or above, and (b) a diluent boiling below 3-chloropropyne, is used.

5. The method according to claim 4 wherein the diluent (a) used, which boils at the same temperature as 3-chloropropyne or above, is an unbranched or branched C₆- to C₈-alkane, an unbranched or branched C₆- to C₈-cycloalkane with a 5-membered or 6-membered ring, benzene, toluene, ethylbenzene, xylenes or mixtures thereof, and the diluent (b) used, which boils below 3-chloropropyne, is a C₃- to C₅-alkane, cyclopentane, an aliphatic ether having a total of 2 to 5 carbon atoms, chloromethane or mixtures thereof.

6. The method according to any of claims 1 to 5 wherein the pressure balance between the total system pressure and the partial pressure of 3-chloropropyne and the partial pressure of the diluent is compensated by the presence of an inert gas.

7. The method according to any of claims 1 to 6 wherein the system is kept at a temperature ranging from 0 to 100°C and at a total pressure ranging from 0.05 to 0.5 MPa abs.

8. The method according to any of claims 1 to 7 wherein the 3-chloropropyne is stored or transported.

9. The method according to any of claims 1 to 7 wherein the 3-chloropropyne is prepared by reacting propyn-3-ol with a chlorinating agent in the presence of a catalyst.

10. The method according to claim 9 wherein the chlorinating agent used is phosgene and the catalyst used is an N,N-disubstituted formamide of the general formula (I): 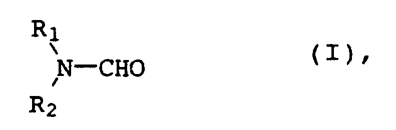 in which R¹ and R² independently of one another are C₁- to C₈-alkyl or R¹ and R² together are a C₄- or C₅-alkylene chain which can optionally be interrupted by one or more oxygen or nitrogen atoms.

11. The use of 3-chloropropyne which has been prepared according to any of claims 1 to 7 and 9 or 10 and/or stored and/or transported according to any of claims 1 to 8 in the synthesis of dyestuffs, pharmaceutical and agricultural active ingredients, electroplating auxiliaries, disinfectants, steroids and growth hormones.

## Revendications

1. Procédé de manipulation intrinsèquement sûre de 3-chloropropyne en présence d'un agent de dilution ayant un point d'ébullition à pression normale de l'ordre de -50°C (223 K) à 200°C (473 K), **caractérisé en ce que** l'on met en oeuvre un agent de dilution ayant un point d'ébullition identique ou supérieur à celui de la 3-chloropropyne, en une quantité de 50 à 2000% en poids dans la phase liquide, par rapport à la quantité de 3-chloropropyne dans la phase liquide, et que l'on maintient la concentration de 3-chloropropyne dans la phase liquide et dans la phase gazeuse, via le type et la quantité de l'agent de dilution, via la température et via la pression totale du système, au-dessous des concentrations susceptibles de déflagration.

2. Procédé selon la revendication 1, **caractérisé en ce que** l'on met en oeuvre l'agent de dilution à point d'ébullition égal ou supérieur à celui de la 3-chloropropyne en une quantité de 50 à 1000% en poids dans la phase liquide, par rapport à la quantité de 3-chloropropyne dans la phase liquide.

3. Procédé selon les revendications 1 à 2, **caractérisé en ce que** l'on met en oeuvre en tant qu'agent de dilution à point d'ébullition égal ou supérieur à celui de la 3-chloropropyne un alcane non ramifié ou ramifié en C₆ à C₈, un cycloalcane non ramifié ou ramifié en C₆ à C₈ à noyau pentagonal ou hexagonal, du benzène, du toluène, de l'éthylbenzène, des xylènes, ou des mélanges d'entre eux.

4. Procédé selon la revendication 1, **caractérisé en ce que** l'on met en oeuvre un mélange constitué de (a) un agent de dilution à point d'ébullition égal ou supérieur à celui de la 3-chloropropyne et (b) un agent de dilution d'un point d'ébullition inférieur à celui de la 3-chloropropyne.

5. Procédé selon la revendication 4, **caractérisé en ce que** l'on met en oeuvre (a) en tant qu'agent de dilution à point d'ébullition égal ou supérieur à celui de la 3-chloropropyne un alcane non ramifié ou ramifié en C₆ à C₈, un cycloalcane non ramifié ou ramifié en C₆ à C₈ à noyau pentagonal ou hexagonal, du benzène, du toluène, de l'éthylbenzène, des xylènes, ou des mélanges d'entre eux et (b) en tant qu'agent de dilution à point d'ébullition inférieur à celui de la 3-chloropropyne un alcane en C₃ à C₅, du cyclopentane, un éther aliphatique comportant au total de 2 à 5 atomes de carbone, du chlorométhane, ou des mélanges d'entre eux.

6. Procédé selon les revendications 1 à 5, **caractérisé en ce que** l'on équilibre le bilan de pression entre la pression totale du système et la pression partielle de la 3-chloropropyne, ainsi que la pression partielle de l'agent de dilution, par la présence d'un gaz inerte.

7. Procédé selon les revendications 1 à 6, **caractérisé en ce que** l'on maintient le système à une température de l'ordre de 0 à 100°C et à une pression totale de l'ordre de 0,05 à 0,5 MPa abs.

8. Procédé selon les revendications 1 à 7, **caractérisé en ce que** l'on entrepose ou transporte la 3-chloropropyne.

9. Procédé selon les revendications 1 à 7, **caractérisé en ce que** l'on prépare la 3-chloropropyne par réaction de propyn-3-ol avec un agent de chloration en présence d'un catalyseur.

10. Procédé selon la revendication 9, **caractérisé en ce que** l'on met en oeuvre en tant qu'agent de chloration du phosgène et en tant que catalyseur un formamide N,N-disubstitué de la formule générale (I) 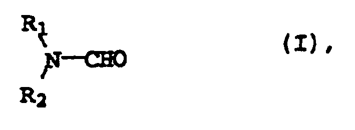 dans laquelle R¹ et R² représentent indépendamment l'un de l'autre des radicaux alkyle en C₁ à C₈, ou bien R¹ et R² représentent ensemble une chaîne alkylène en C₄ ou en C₅, qui peut le cas échéant être interrompue par un ou plusieurs atomes d'oxygène ou d'azote.

11. Utilisation de 3-chloropropyne, préparée selon les revendications 1 à 7 et 9 à 10 et/ou entreposée et/ou transportée selon les revendications 1 à 8, dans la synthèse de colorants, de produits pharmaceutiques et agricoles, d'adjuvants de galvanisation, de désinfectants, de stéroïdes et d'hormones de croissance.
